# FASCICULE DE BREVET EUROPEEN

(11) **EP 2 046 429 B1**
(45) Date de publication et mention de la délivrance du brevet: **20.06.2018**
(21) Numéro de dépôt: 07823591.8
(22) Date de dépôt: 17.07.2007
(51) Int. Cl.: A61M 15/00

(54) **DISPOSITIF DE DISTRIBUTION DE PRODUIT FLUIDE**
SPENDER FÜR EIN FLÜSSIGPRODUKT
FLUID-PRODUCT DISPENSING DEVICE

(30) Priorité: 25.07.2006 FR 0653105
(43) Date de publication de la demande: 15.04.2009
(73) Titulaire: Aptar France SAS, 27110 Le Neubourg (FR)
(72) Inventeur: POCOCK, Andrew Gordon, Ickleton Cambridgeshire CB10 1SX (GB); KAY, Stuart Brian William, Ickleton Cambridgeshire CB10 1SX (GB); GREENHALGH, Paul, Ickleton Cambridgeshire CB10 1SX (GB); O'HARA, Wayne, Ickleton Cambridgeshire CB10 1SX (GB)
(74) Mandataire: CAPRI
(86) Numéro de dépôt international: PCT/FR2007/051671
(87) Numéro de publication internationale: WO 2008/012457

(56) Documents cités:
- WO-A-01/26720
- WO-A-90/13328
- WO-A-03/035508
- WO-A-2004/067069
- WO-A-2006/079750

## Description

La présente invention concerne un dispositif de distribution de produit fluide, et plus particulièrement un inhalateur de poudre sèche.

Les inhalateurs de poudre sèche sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Un autre type d'inhalateur consiste à emballer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blister allongé ou des blisters disposés sur un disque circulaire rotatif. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre évidemment, l'utilisation est plus complexe, puisque l'utilisateur est obligé de charger une capsule dans l'inhalateur avant chaque utilisation. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a changé la dose (soit à partir d'un réservoir multidoses soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. En variante, il a été proposé de percer la paroi ou couche de fermeture. Ceci présente l'inconvénient que les parties de paroi découpées risquent de retenir une partie de la dose à l'intérieur du réservoir, la précision et reproductibilité de dosage n'étant donc pas garanties.

Le document WO0126720 décrit un dispositif de l'état de la technique.

La présente invention a pour but de fournir un dispositif de distribution de produit fluide, en particulier un inhalateur de poudre sèche qui ne reproduit pas les inconvénients susmentionnés.

En particulier, la présente invention a pour but de fournir un dispositif qui soit simple et peu coûteux à fabriquer et à assembler, fiable d'utilisation, garantissant une précision de dosage et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a aussi pour but de fournir un dispositif qui évite tout risque de sous-dosage, l'ouverture du réservoir, l'expulsion de la dose et le comptage de la dose émise ne se produisant qu'après l'inhalation de l'utilisateur. De plus, la présente invention a pour but d'éviter tout risque de perte de dose en l'absence d'inhalation même si l'utilisateur manipule le dispositif.

La présente invention a encore pour but de fournir un dispositif permettant de compter le nombre de doses émises ou restant à émettre.

La présente invention a donc pour objet un dispositif de distribution de produit fluide tel que décrit dans la revendication 1. Des modes de réalisation avantageux sont décrits dans les revendications dépendantes.

Ces caractéristiques et avantages et d'autres de la présente invention apparaîtront plus clairement au cours de la description détaillée suivante de plusieurs modes et variantes de réalisation de celle-ci, faite en référence aux dessins joints, donnés à titres d'exemples non limitatifs, sur lesquels,
- la figure 1 est une vue schématique externe d'un dispositif selon une variante avantageuse de l'invention,
- la figure 2 est une vue schématique en section d'un dispositif selon un mode de réalisation de l'invention, en position de fermeture,
- la figure 3 est une vue similaire à la figure 2, en cours d'ouverture,
- la figure 4 est une vue similaire à la figure 3, en position d'ouverture,
- la figure 5 est une vue similaire à la figure 4, après inhalation,
- la figure 6 est une vue similaire à la figure 5, en début de fermeture après inhalation,
- la figure 7 est une vue similaire à la figure 6, en cours de fermeture après inhalation,
- la figure 8 est une vue similaire à la figure 7, en fin de fermeture après inhalation,
- la figure 9 est une vue similaire à la figure 4, en début de fermeture en l'absence d'inhalation,
- la figure 10 est une vue schématique en section d'un dispositif selon un autre mode de réalisation de l'invention, en position de fermeture,
- la figure 11 est une vue similaire à la figure 10, en position d'ouverture,
- la figure 12 est une vue similaire à la figure 11, après inhalation,
- la figure 13 est une vue similaire à la figure 12, en cours de fermeture après inhalation,
- la figure 14 est une vue similaire à la figure 13, en fin de fermeture après inhalation,
- la figure 15 est une vue schématique d'un dispositif selon une variante de réalisation de l'invention, montrant en transparence le dispositif d'inhalation de doses, en position de fermeture,
- la figure 16 est une vue schématique en section du dispositif de la figure 15,
- la figure 17 est une vue en détail de la figure 16,
- la figure 18 est une vue similaire à la figure 15, en position d'ouverture,
- la figure 19 est une vue similaire à la figure 18, après inhalation,
- la figure 20 est une vue similaire à la figure 19, en fin de fermeture après inhalation,
- la figure 21 est une vue schématique en section d'un dispositif selon un autre mode de réalisation de l'invention, en position de fermeture,
- la figure 22 est une vue similaire à la figure 21, en cours d'ouverture,
- la figure 23 est une vue similaire à la figure 22, en position d'ouverture,
- la figure 24 est une vue similaire à la figure 23, après inhalation,
- la figure 25 est une vue similaire à la figure 24, en cours de fermeture après inhalation,
- la figure 26 est une vue similaire à la figure 25, en fin de fermeture après inhalation,
- les figures 27a et 27b sont des vues schématiques en section, respectivement de devant et de derrière, d'un autre mode de réalisation de l'invention, en position de fermeture ;
- les figures 28a et 28b sont des vues similaires aux figures 27a et 27b, en position d'ouverture,
- les figures 29a et 29b sont des vues similaires aux figures 28a et 28b, après inhalation,
- les figures 30a et 30b sont des vues similaires aux figures 29a et 29b, en cours de fermeture après inhalation,
- les figures 31a et 31b sont des vues similaires aux figures 30a et 30b, en fin de fermeture après inhalation,
- la figure 32 est une vue similaire à la figure 5, montrant schématiquement le support de réservoirs et les moyens d'ouverture selon une variante avantageuse de l'invention

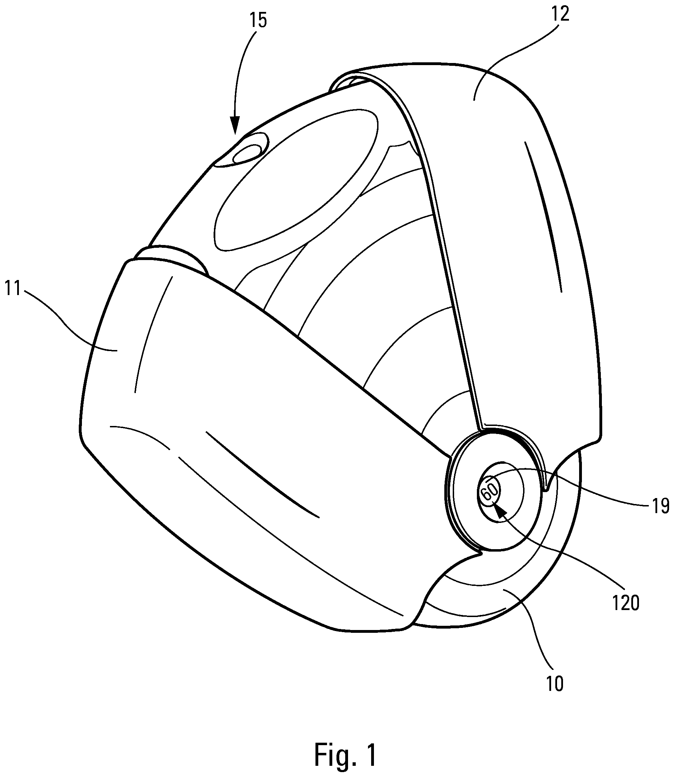

La figure 1 représente une vue externe d'un mode de réalisation d'un inhalateur de poudre sèche. Cet inhalateur comporte un corps central 10 sur lequel sont montées coulissantes deux éléments ou ailes latérales 11, 12 formant capot lorsque le dispositif est fermé et adaptées à être séparées l'une de l'autre pour ouvrir le dispositif et ainsi charger le dispositif comme cela sera décrit ci-après. Le corps 10 peut être de forme environ arrondie en partie basse, et relativement plat en partie haute, comme représenté sur la figure 1, mais il pourrait avoir tout autre forme appropriée. Le corps 10 comporte un orifice de distribution et d'inhalation 15 à travers lequel l'utilisateur va inhaler lors de l'actionnement du dispositif. Les deux parties latérales 11, 12 formant capot peuvent s'ouvrir par pivotement autour d'un axe de rotation commun comme représenté sur la figure 1, mais tout autre moyen d'ouverture du dispositif est envisageable. Par ailleurs, on pourrait ne prévoir qu'un seul élément de capot mobile par rapport au corps au lieu des deux représentés sur la figure 1.

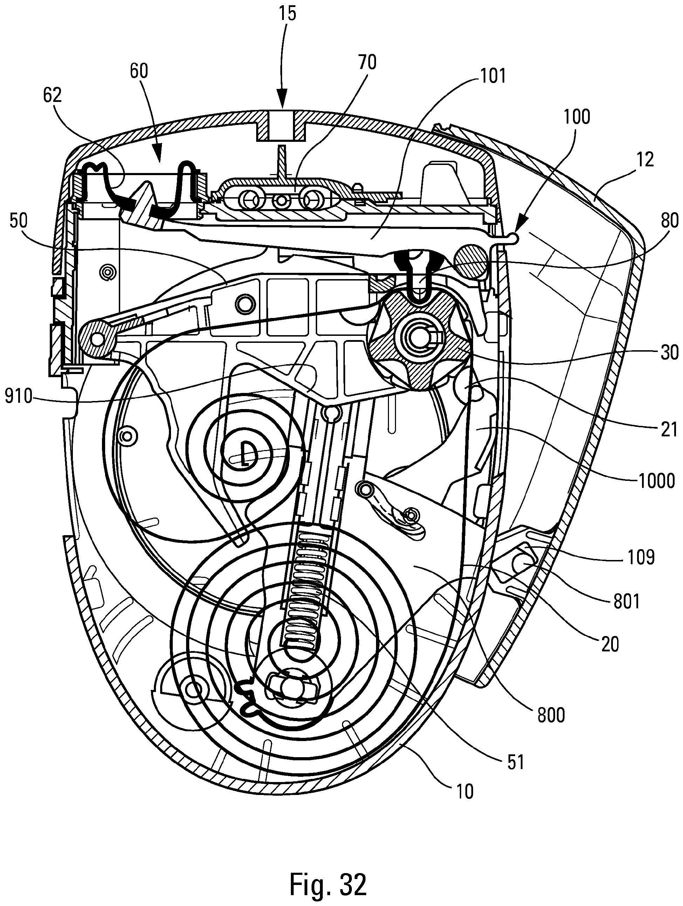

Avantageusement, le corps comporte une fenêtre 19 à travers laquelle le comptage des doses émises ou restant à émettre peut être affiché de manière visible pour l'utilisateur. Cette fenêtre 19 peut avantageusement être prévue sur ou proche de l'axe de rotation des éléments de capot 11, 12 formant le capot. A l'intérieur du corps, il peut être prévu un support 20 de réservoirs individuels 21. Les réservoirs sont avantageusement du type blisters, et le support de réservoir est de préférence une bande allongée, sur laquelle les blisters sont disposés les uns derrière les autres, de manière connue. Cette bande et ces blisters ne sont représentés que partiellement sur la figure 32 pour ne pas surcharger les autres dessins dans des buts de clarté. La bande de blister peut avantageusement être constituée d'une couche ou paroi de base formant des cavités recevant les doses de poudre, et d'une couche ou paroi de fermeture qui obture de manière étanche chacun desdits blisters. La bande de blister peut être enroulée à l'intérieur du corps et des moyens d'entraînement de bande 30 sont prévus pour progressivement dérouler cette bande de blister et amener un réservoir individuel ou blister respectif dans une position de distribution à chaque actionnement du dispositif. Lorsqu'un réservoir individuel a été vidé par une inhalation, la partie de bande comportant lesdits réservoirs vides est avantageusement adaptée à s'enrouler dans un autre endroit dudit corps 10.

Des moyens d'ouverture de réservoir 80 sont prévus dans ou solidaires du corps 10, ces moyens d'ouverture comportant des moyens de perçage et/ou de coupage de la couche de fermeture des blisters. Ces moyens d'ouverture ne sont également représentés que schématiquement sur la figure 32 pour ne pas surcharger les autres dessins dans des buts de clarté.

Des moyens de support mobiles 50 sont adaptés à supporter au moins le réservoir qui est destiné à être ouvert lors de la prochaine inhalation. Ces moyens de supports mobiles 50 sont adaptés à déplacer le réservoir à vider contre lesdits moyens d'ouverture du dispositif lors de l'actionnement. Avantageusement, ces moyens de supports mobiles 50 sont sollicités par un élément de chargement élastiquement déformable, tel qu'un ressort, une tige ou tout autre élément élastique équivalent, ledit élément de chargement pouvant être préchargé, notamment lors de l'ouverture du dispositif. Avantageusement, les moyens de support mobiles 50 sont déplaçables entre une première position (position de non-distribution) et une seconde position (position de distribution) qui est la position d'ouverture de réservoir.

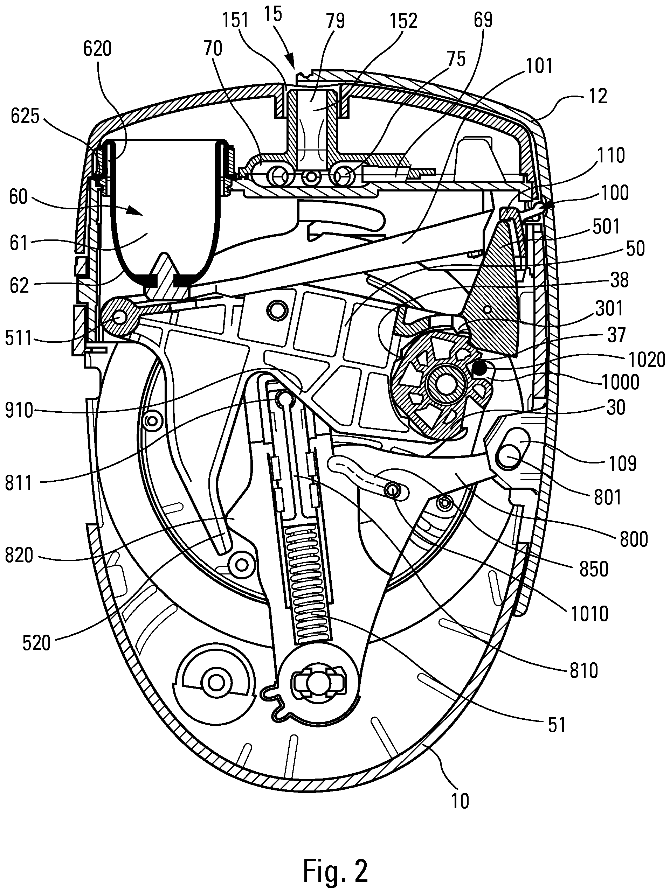

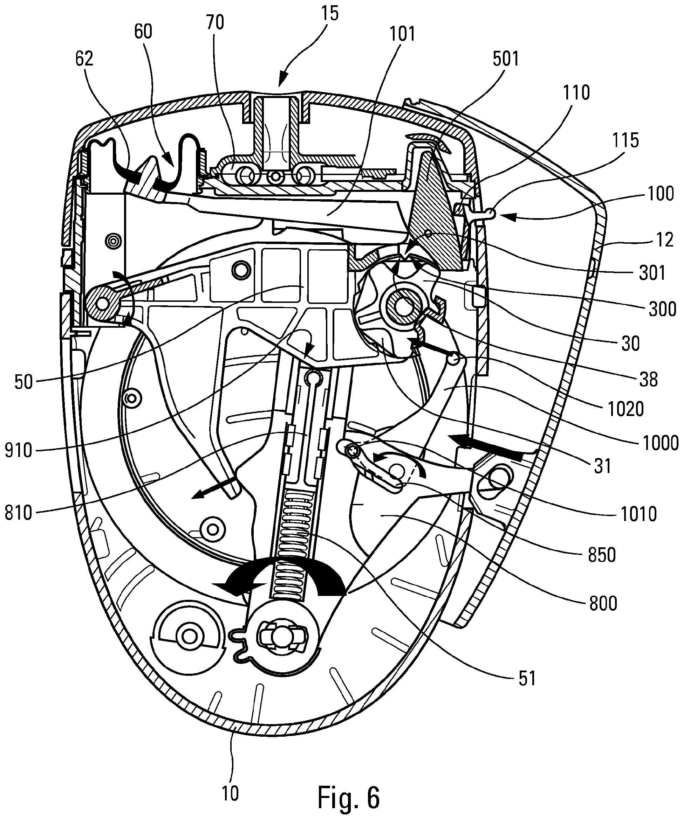

Avantageusement, les moyens de support mobiles 50 comportent une pièce sensiblement rigide, telle qu'une tige, articulée par rapport audit corps 10. Une roue de guidage 30 fixée de manière rotative sur lesdits moyens de support mobiles 50, reçoit et guide les blisters. Une rotation de cette roue de guidage 30 fait ainsi avancer la bande de blister dans une première direction. Dans une position angulaire particulière, un réservoir ou blister donné est toujours en position pour être ouvert par les moyens d'ouverture. Avantageusement, des moyens de positionnement en rotation 300 de ladite roue de guidage 30 peuvent être prévus pour déterminer précisément la position angulaire de ladite roue de guidage 30 après chaque rotation. Ces moyens de positionnement 300 peuvent, selon une variante avantageuse, comporter une projection ou doigt 301 dont une extrémité coopère élastiquement avec des encoches 38 prévues autour de ladite roue de guidage 30. Avantageusement, les encoches 38 comportent un profil environ en V qui guide automatiquement ledit doigt 301 vers la position centrale de l'encoche, assurant un positionnement angulaire précis à chaque rotation. Ces moyens de positionnement 300 sont notamment visibles sur les figures 2 et 6. La roue de guidage 30 forme de préférence les seuls moyens d'entraînement du support de réservoir. Eventuellement, une (ou plusieurs) roue complémentaire pourrait être prévue pour aider au guidage et/ou à l'entraînement du support de réservoirs.

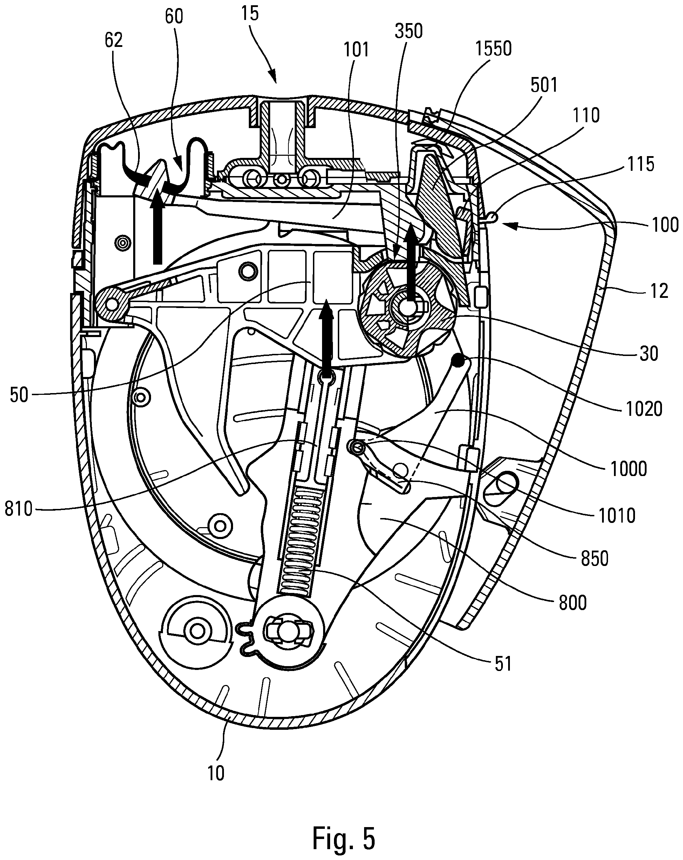

Avantageusement, des moyens de butée 350 sont prévus pour déterminer précisément la position de distribution de la roue de guidage 30 lors de chaque inhalation. Ces moyens de butée peuvent comporter un plot 350 adapté à coopérer en position de distribution avec une ou des surface(s) plane(s) correspondante(s) de la roue de guidage 30. De préférence, une surface plane est associée à chaque évidement. Dans cet exemple, cette butée 350 participe au positionnement correct en rotation de la roue de guidage 30 au moment où les moyens d'ouverture, notamment les moyens de perçage et/ou coupage, pénètrent dans le réservoir à vider. La butée 350 définit donc non seulement la profondeur de pénétration desdits moyens de perçage et/ou coupage dans le réservoir, mais aussi leur centrage par rapport au réservoir, afin de garantir un expulsion optimale de la poudre et une reproductibilité de dose élevée à chaque actionnement. Ces moyens de butée 350 peuvent être associés aux moyens de positionnement en rotation 300 susmentionnés de manière à prédéterminer de manière précise chaque position de la roue de guidage, en position de non-distribution, en position de distribution, et aussi lors des déplacements de la roue de guidage 30 entre ces positions. Ceci permet d'éviter des risques de blocage du dispositif en cas de mauvais positionnement de ladite roue de guidage. Ces moyens de butée 350 apparaissent notamment sur la figure 5.

Lorsque le réservoir se déplace vers sa position d'ouverture pour être ouvert par les moyens d'ouverture 80, ces moyens d'ouverture sont de préférence fixes par rapport au corps 10. Toutefois, il est envisageable que ces moyens de d'ouverture puissent également être mobiles pendant la phase d'ouverture du réservoir. Par exemple, les moyens d'ouverture pourraient se déplacer en direction du réservoir pendant que le réservoir se déplace en direction des moyens d'ouverture. Dans une autre variante, on pourrait également envisager que le réservoir et les moyens d'ouverture se déplacent dans la même direction lors de l'actionnement, le réservoir se déplaçant plus rapidement dans cette direction de sorte qu'il vient en contact avec lesdits moyens d'ouverture pour être ouvert.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture de réservoir, on peut prévoir un système de déclenchement par l'inhalation qui comporte avantageusement des moyens 60 déplaçables et/ou déformables sous l'effet de l'inhalation, ces moyens 60 étant adaptés à libérer des moyens de blocage 100. Ces moyens 60 comprennent avantageusement une chambre d'air déformable 61 coopérant avec les moyens de blocage 100 desdits moyens de support mobiles 50. L'inhalation de l'utilisateur provoque la déformation de ladite chambre d'air déformable 61, permettant ainsi de libérer lesdits moyens de blocage 100 et donc de débloquer les moyens de support mobiles 50 pour permettre le déplacement de la roue de guidage 30, et donc du réservoir à vider, vers sa position d'ouverture. Avantageusement, cette chambre d'air 61 peut comprendre une membrane déformable 62, qui peut être reliée d'une part à l'orifice d'inhalation 15 et d'autre part auxdits moyens de blocage 100 de manière directe ou indirecte. Ainsi, lors de l'inhalation, la membrane 62 se déforme et/ou se contracte, provoquant ainsi le déplacement desdits moyens de blocage 100 dans une position de déblocage. Avantageusement, une poche ou diaphragme 62 peut former la chambre d'air 61. Cette poche 62 est reliée à l'orifice d'inhalation 15 via un canal 151 avantageusement disposé autour du canal d'expulsion 152 relié à une chambre de distribution 70. La poche 62 peut être fixée à une tige 101 reliée aux moyens de blocage 100, l'inhalation provoquant la déformation de la poche 62 et donc le pivotement de la tige 101 pour déplacer lesdits moyens de blocage 100. Avantageusement, la poche 62 peut être réalisée en silicone, et comporter un ourlet 620 adapté à former une étanchéité avec le corps 10. Pour ce faire, l'ourlet 620 peut se prolonger par une bride 625, également en silicone, qui va être comprimée par un partie d'encliquetage du corps 10 pour réaliser l'étanchéité, et notamment éviter toute perte de charge dans l'écoulement d'inhalation. En variante, la chambre d'air déformable pourrait être réalisée différemment, notamment par une membrane déformable quelconque.

L'inhalateur comporte en outre une chambre de distribution 70 qui est destinée à recevoir la dose de poudre après ouverture d'un réservoir respectif. Avantageusement, cette chambre de distribution 70 est pourvue d'au moins un élément sensiblement sphérique 75, tel qu'une bille, qui se déplace à l'intérieur de ladite chambre 70 pendant l'inhalation, pour améliorer la distribution du mélange air et poudre après ouverture d'un réservoir, afin d'augmenter l'efficacité du dispositif.

Selon une variante particulière, la chambre d'air déformable 61 coopère avec la chambre de distribution 70. Cette chambre de distribution 70 peut donc être reliée aux moyens d'ouverture du réservoir, et en particulier aux moyens de perçage et/ou de coupage, et comporte un orifice de distribution 79, avantageusement relié directement à l'orifice de distribution et d'inhalation 15 du dispositif. La membrane 62 peut donc être connectée d'une part à l'orifice d'inhalation 15, et d'autre part à cette chambre de distribution 70, dans le chemin d'écoulement d'inhalation de l'utilisateur. Il peut être avantageux que les moyens d'ouverture, en particulier les moyens de perçage et/ou de coupage soient formés directement sur ladite chambre de distribution 70, par exemple à l'extrémité d'un canal 69 menant à ladite chambre 70.

Après l'inhalation, lorsque l'utilisateur referme le dispositif, tous les composants reviennent vers leur position de repos initiale, à savoir que les moyens de support mobiles 50 pivotent par rapport à leur axe de pivotement en retour vers leur position de non distribution en éloignement des moyens d'ouverture de réservoir, et l'élément de chargement est également ramené dans sa position de repos initiale dans laquelle il n'est pas comprimé ou déformé. Le dispositif est alors prêt pour un nouveau cycle d'utilisation.

D'autres moyens de déclenchement par l'inhalation pourraient aussi être utilisés en variante, par exemple en utilisant un clapet pivotant qui, lorsque l'utilisateur inhale, pivote sous l'effet de la dépression créée par cette inhalation, le pivotement de ce clapet provoquant la libération des moyens de blocage des moyens de support mobiles et donc le déplacement du réservoir vers les moyens d'ouverture.

Avantageusement, les moyens de support mobiles 50 qui supportent la roue de guidage 30 peuvent comporter une extension 501, indiquée sur les figures 2 et 5, qui sert notamment à coopérer avec les moyens de blocage 100. Cette extension peut en outre également servir à sensiblement boucher un trou 1550 prévu dans le dispositif, lorsque les moyens de support mobiles sont en position de distribution. Ainsi, en position de non distribution, l'écoulement d'inhalation passe en partie par le trou 1550. Après déplacement des moyens de support mobiles 50 vers la position de distribution, ce qui provoque sensiblement le bouchage du trou 1550, et l'ouverture du réservoir, l'écoulement d'inhalation est principalement canalisé vers ledit réservoir ouvert. Il s'en suit une meilleure efficacité lors de l'inhalation qui participe à assurer un vidage optimal du réservoir.

Selon un autre aspect avantageux de l'inhalateur, les réservoirs individuels ou blisters 21 sont formés sur une bande allongée 20, qui est stockée sous forme de bobine à l'intérieur du corps 10 du dispositif. Avantageusement, cette bande de blister enroulée est maintenue par des parois internes dudit corps 10 sans que son extrémité « arrière » (dans le sens de déplacement de la bande de blisters) ne soit fixée par rapport audit corps 10, ce qui permet un assemblage plus aisé de cette bande de blister à l'intérieur du dispositif. La bande de blister est déplacée avantageusement au moyen de la roue de guidage 30 qui présente avantageusement au moins un, de préférence plusieurs évidements 31, visibles sur la figure 6, dont la forme correspond sensiblement à celle des blisters. Ainsi, lorsque cette roue de guidage 30 tourne, elle entraîne la bande de blister dans la première direction. Bien entendu en variante ou de manière additionnelle, on pourrait utiliser d'autres moyens d'avancée de bande de blisters, par exemple en prévoyant un profil sur les bords longitudinaux latéraux de la bande de blister, ledit profil étant adapté à coopérer avec des moyens d'entraînement appropriés. De même, des trous ménagés le long des bords latéraux de la bande de blister pourraient également être utilisés pour faire avancer la bande de blister au moyen de roues dentées coopérant avec lesdits trous, à la manière d'une pellicule photo.

Selon encore un autre aspect de l'inhalateur, un dispositif de comptage ou d'indication de doses est également prévu. Ce dispositif peut comporter des chiffres ou symboles inscrits directement sur la bande de blister et visibles à travers une fenêtre appropriée 19 dans le corps 10 du dispositif. En variante, on pourrait envisager d'utiliser un ou plusieurs disque(s) rotatif(s) comportant les chiffres ou symboles, comme cela sera décrit ci-après.

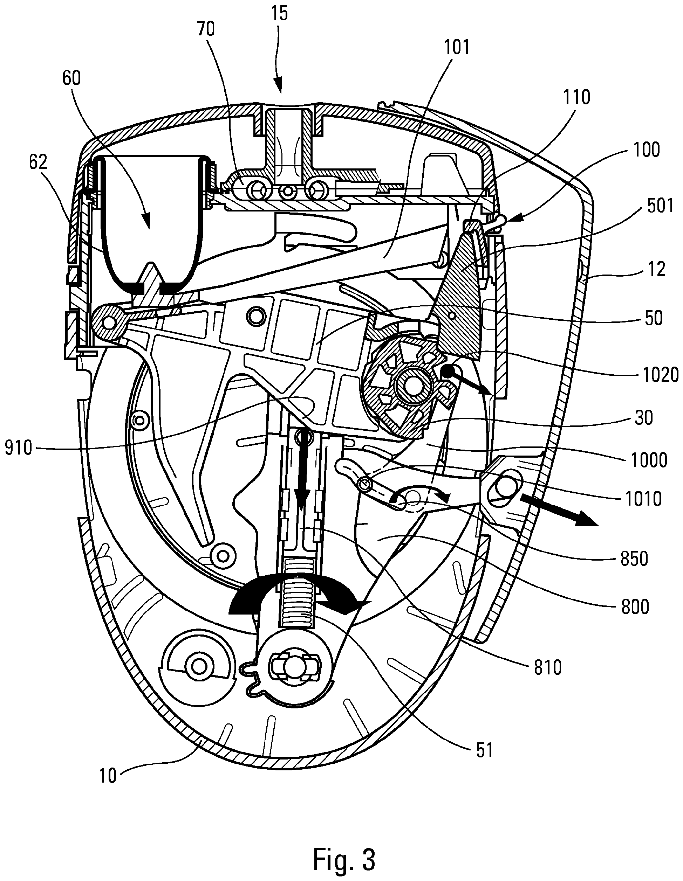

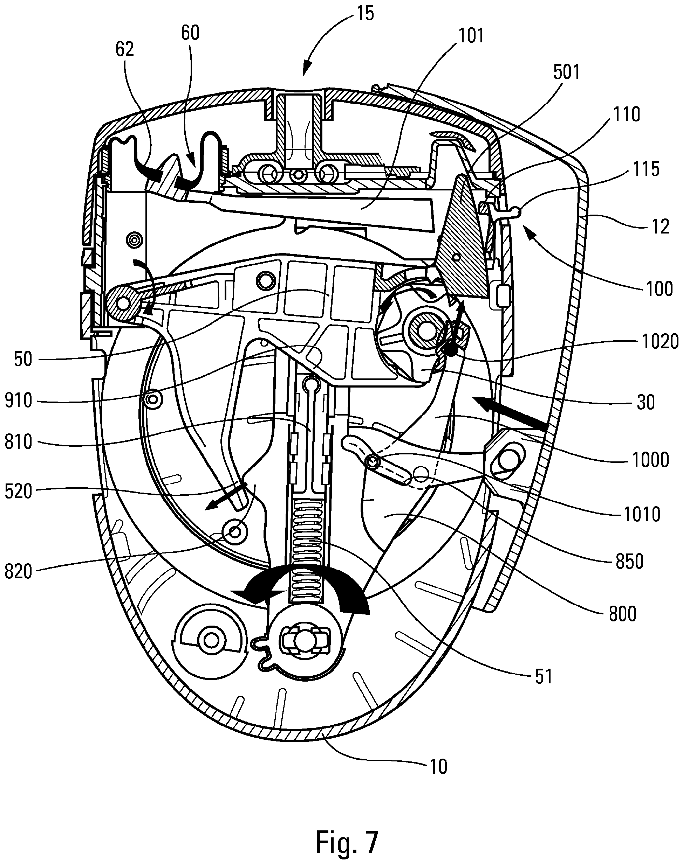

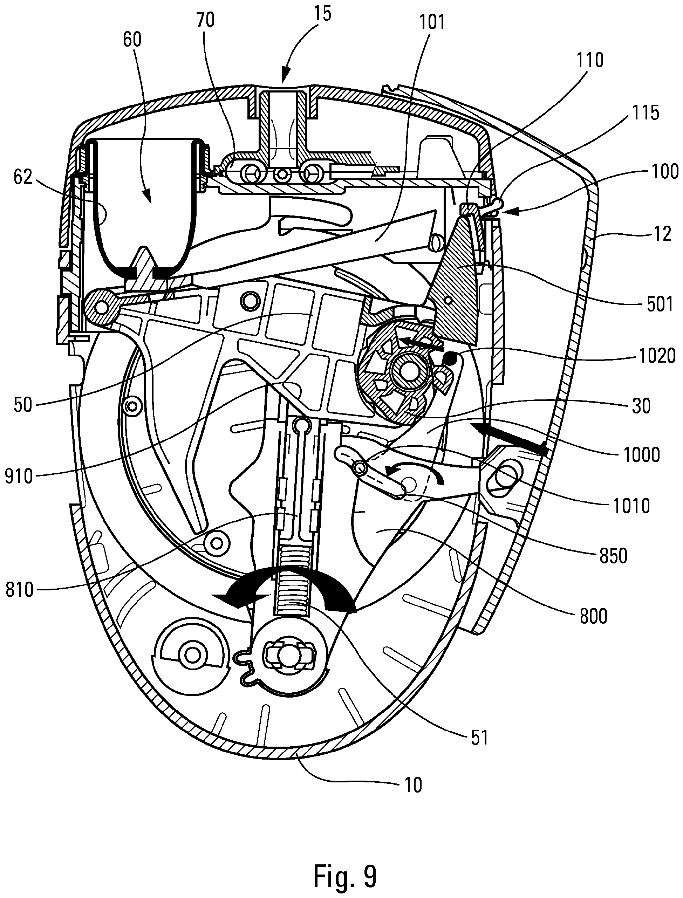

Les figures 2 à 9 représentent un mode de réalisation avantageux de la présente invention. En référence à ce mode de réalisation, le dispositif comporte un corps 10 sur lequel sont articulés deux éléments de capot 11, 12 autour avantageusement d'un axe d'articulation commun, comme cela est visible sur la figure 1. Pour des raisons de clarté, un seul des éléments de capot, en l'occurrence celui représenté sur le côté droit du dispositif et indiqué par la référence numérique 12, est représenté sur les figures. Les déplacements des différentes pièces sont indiqués schématiquement par des flèches sur certaines figures.

Ledit élément de capot mobile 12 est connecté à un organe d'armement 800, avantageusement par l'intermédiaire d'une ouverture 109 qui peut être de forme oblongue dans laquelle vient se placer un ergot ou similaire 801 dudit organe d'armement 800. Cet organe d'armement 800 est avantageusement monté pivotant sur le corps 10 autour d'un axe de pivotement. Cet organe d'armement 800 supporte l'élément de chargement 51 qui, dans ce mode de réalisation, est réalisé sous la forme d'un ressort à boudins. Ce ressort 51 coopère avec une tige 810, connectée d'un côté audit ressort à boudin 51 et de l'autre côté à une surface de came 910 prévue sur lesdits moyens de support mobiles 50, qui sera décrite ultérieurement. La tige 810, lorsque l'organe d'armement 800 est déplacé autour de son axe de pivotement lors du déplacement de l'élément de capot mobile 12, est donc adaptée à comprimer le ressort 51 lorsque l'élément de capot 12 est ouvert, et à décomprimer ledit ressort 51 lorsque ledit élément de capot 12 est refermé. Avantageusement, la tige 810 comporte, dans sa partie en contact avec la surface de came 910, une partie arrondie 811, telle qu'une extrémité en forme de boule, pour favoriser le glissement de la tige 810 sur ladite surface de came 910. L'organe d'armement 800 comporte en outre une projection 820 adaptée à coopérer avec une partie d'extension 520 desdits moyens de support mobiles 50, comme cela sera décrit plus en détail ci-après. L'organe d'armement 800 comporte en outre des moyens de guidage 850, avantageusement formés sous la forme d'une rainure, dans laquelle est reçu une projection 1010 solidaire d'un élément d'entraînement 1000, qui sera également décrit plus en détail ci-après. Avantageusement, ladite rainure 850 comporte au moins deux parties de pente différentes, dont la fonction sera également décrite ultérieurement.

Les moyens de support mobiles 50 sont dans ce mode de réalisation réalisés sous la forme d'une pièce montée pivotante sur le corps autour d'un axe de pivotement 511. Ces moyens de support mobiles 50 incorporent également une extension 501, avantageusement en forme d'aileron. La surface de came précitée 910 est formée sur lesdits moyens de support mobiles 50 de sorte que lorsque le ressort 51 est chargé lors de l'ouverture de l'élément de capot mobile 12, lesdits moyens de support mobiles 50 sont sollicités vers leur position de distribution par ladite tige 810 poussée par le ressort 51 comprimé. Des moyens de blocage 100 sont prévus pour retenir lesdits moyens de support mobiles 50 dans ladite position de non distribution, représentée notamment sur la figure 2. Lesdits moyens de blocage 100 comportent avantageusement un élément de blocage 110 adapté à coopérer avec ladite extension 501 desdits moyens de support mobiles 50. Lesdits moyens de blocage 100 sont avantageusement connectés au diaphragme déformable 62 sensible à l'inhalation de l'utilisateur par l'intermédiaire d'une tige 101, de sorte que lors de l'inhalation de l'utilisateur, ledit diaphragme se déforme, faisant ainsi pivoter la tige 101 et par la même ledit élément de blocage 110, libérant ainsi l'extension 501. Ceci permet le déplacement desdits moyens de support mobiles 50 vers leur position de distribution sous l'effet de la force exercée par le ressort comprimé 51. Ce déplacement des moyens de support mobiles 50 provoque l'ouverture d'un réservoir individuel comme décrit précédemment.

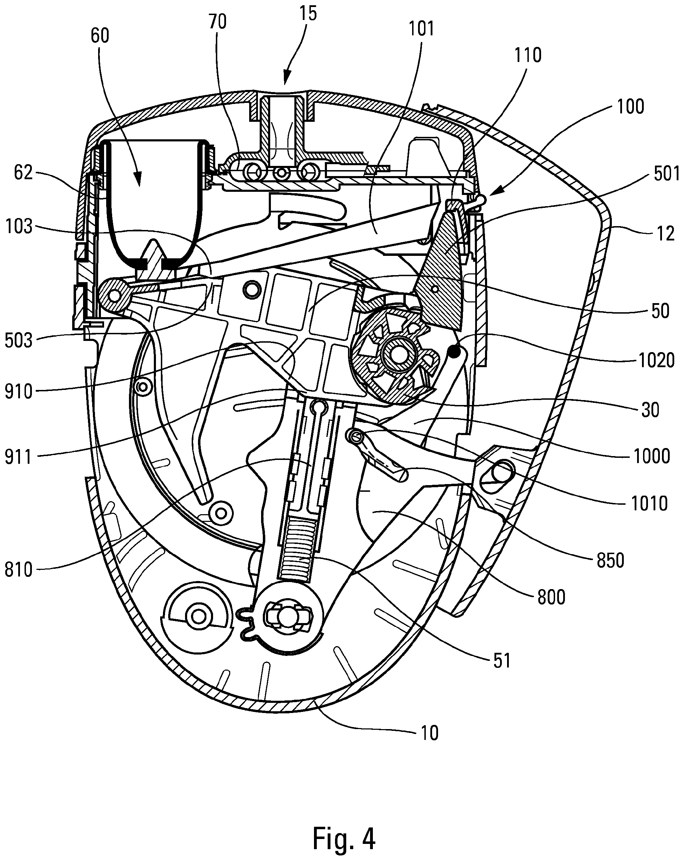

L'élément d'entraînement 1000 est avantageusement monté pivotant à l'intérieur du corps 10. Comme expliqué précédemment, cet élément d'entraînement 1000 coopère d'une part avec la rainure 850 de l'organe de chargement 800 par l'intermédiaire de sa projection 1010. Par ailleurs, une autre projection 1020 dudit élément d'entraînement 1000 coopère avec une denture 37 de la roue de guidage 30. Lorsque l'élément de capot 12 est en position fermée, la projection 1020 est engrenée à l'intérieur de ladite denture 37. Au moment de l'ouverture de l'élément de capot 12, la projection 1010 de l'élément d'entraînement coulisse dans la rainure 850 de l'organe de chargement provoquant le pivotement dudit élément d'entraînement 1000 autour de son axe de pivotement. Ce pivotement fait sortir la projection d'entraînement 1020 de ladite denture 37 de la roue de guidage 30. Cette position désengagée est représentée sur la figure 4. Lorsque l'utilisateur inhale, les moyens de support mobiles 50 se déplacent vers la position de distribution. La roue de guidage 30 étant fixée de manière rotative sur lesdits moyens de support mobiles 50, elle se déplace évidemment ensemble avec ceux-ci en direction des moyens d'ouverture. Dans la position de distribution, la projection d'entraînement 1020 de l'élément d'entraînement 1000 se trouve donc face à une autre dent de la denture 37 de la roue de guidage 30, comme cela est clairement visible sur la figure 5. Lorsque l'utilisateur referme ensuite l'élément de capot 12, la projection 1010 de l'élément d'entraînement coulisse à nouveau dans l'autre sens dans la rainure 850 de l'organe de chargement 800, provoquant le pivotement en retour dudit élément d'entraînement 1000. Ainsi, la projection d'entraînement 1020 vient s'engrener dans l'autre dent, notamment la prochaine, de la denture 37 de la roue de guidage 30, comme cela apparaît sur les figures 6 à 8. Cet engrènement se produit avantageusement au début, notamment dans la première moitié, de la course de retour de l'élément de capot mobile 12 vers sa position de fermeture, et la poursuite de la course de retour provoque la rotation de la roue de guidage 30 sous l'effet dudit élément d'entraînement 1000. De cette manière, la roue de guidage 30 tourne autour de son axe de rotation pour amener le prochain blister plein en face des moyens d'ouverture en vue du prochain actionnement du dispositif et donc de la prochaine distribution d'une dose. Comme expliqué précédemment, la rainure 850 de l'organe de chargement comporte avantageusement deux parties de pente différentes. En partant de la position fermée de l'élément de capot mobile, la première partie de la rainure 850 ne provoque avantageusement pas de pivotement de l'élément d'entraînement 1000 et ce n'est que dans la deuxième partie de pente supérieure de la rainure 850 que l'élément d'entraînement 1000 est amené à pivoter autour de son axe de pivotement pour sortir de la denture 37 de l'élément d'entraînement. Par conséquent, lorsque l'utilisateur referme le capot, la projection 1020 d'entraînement de l'élément d'entraînement 1000 revient rapidement à l'intérieur de la prochaine dent de la denture 37, et la poursuite de la course de retour de l'élément de capot mobile vers sa position de fermeture provoque la rotation de ladite roue de guidage 30.

La surface de came 910 comporte également au moins deux parties de pente différentes, avantageusement séparées par un sommet 911. En partant à nouveau de la position fermée de l'élément de capot mobile, la première partie de pente sur laquelle coulisse la tige 810 permet la compression du ressort 51 comme décrit précédemment. Lorsque le ressort est chargé, c'est-à-dire comprimé, la surface de came 910 prévoit une seconde partie de pente différente avec laquelle la tige 810 va coopérer en position d'ouverture du dispositif. De préférence, la tige 810 exerce une force sensiblement perpendiculaire sur cette seconde partie de surface de came, comme visible sur la figure 4. De cette manière, la position chargée est stable.

En position d'ouverture, représentée sur la figure 4, les moyens de support mobiles 50 qui sont sollicités vers la position de distribution par le ressort comprimé 51 exercent donc une force sur les moyens de blocage 100, en particulier sur l'élément de blocage 110 de ces moyens de blocage par l'intermédiaire de l'extension 501. Avantageusement, du côté opposé de la tige 101, à proximité de la connexion de ladite tige 101 au diaphragme 62, il est prévu une zone d'appui 103 adaptée à coopérer avec une zone complémentaire 503 prévue sur les moyens de support mobiles 50. Cette zone d'appui 103 permet de créer une position chargée stable entre lesdits moyens de support mobiles 50 et lesdits moyens de blocage 100. En effet, ces deux moyens sont chacun mobiles et le double contact, avec d'une part une force exercée vers le haut (en se référant à la position représentée sur la figure 4) par l'extension 501 sur la partie d'épaulement 110, et d'autre part une force exercée vers le bas par la zone d'appui 103 sur la zone complémentaire 503, garantit un équilibre de ce blocage qui ne peut être libéré que par l'inhalation de l'utilisateur provoquant la déformation du diaphragme 62 et donc le pivotement de la tige 101 des moyens de blocage 100.

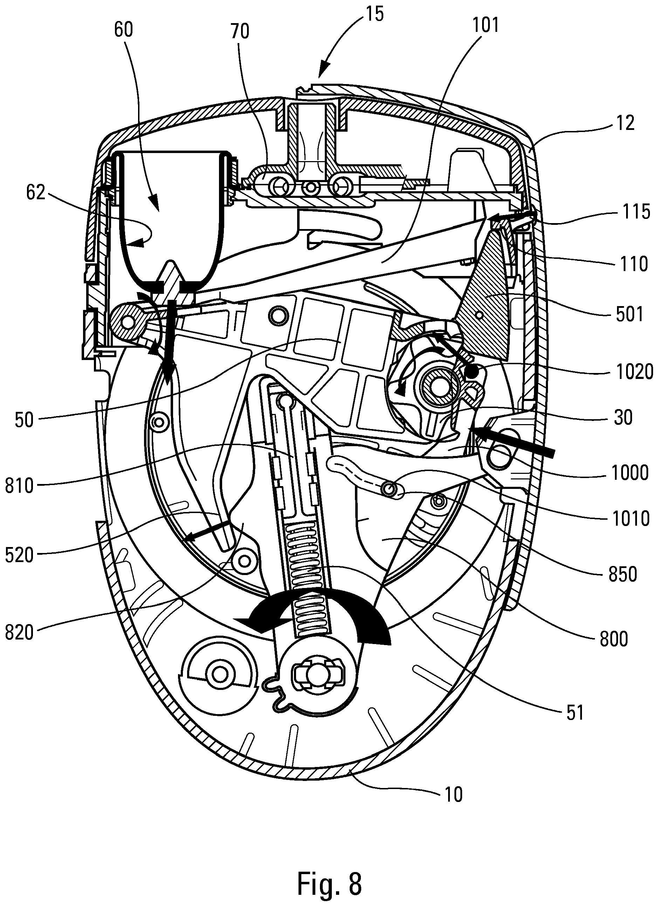

Après l'inhalation, c'est-à-dire en position de distribution représentée sur la figure 5, les moyens de blocage 100 ont pivoté et les moyens de support mobiles 50 ont été déplacés vers le haut par le ressort comprimé 51. Le pivotement des moyens de blocage, en particulier de l'élément de blocage 110, provoque la sortie hors du corps 10 d'une partie d'extrémité 115 de cet élément de blocage 110, comme visible notamment sur la figure 5. Lorsque l'utilisateur referme ensuite l'élément de capot mobile 12, ledit élément de capot 12 vient en fin de fermeture pousser sur ladite partie d'extrémité 115 ce qui ramène les moyens de blocage 100 vers leur position initiale avec le diaphragme 62 qui est également ramené dans sa position initiale, comme visible notamment sur la figure 8.

Ainsi, lorsque l'utilisateur ouvre l'inhalateur il charge le système. S'il n'inhale pas et qu'il referme l'inhalateur, celui-ci reprend simplement sa position de départ sans déplacer les réservoirs 21 ni les moyens de blocage 100. Il n'y a donc aucun risque qu'un réservoir (et donc une dose de produit actif) soit perdu par un actionnement malencontreux ou incomplet dans lequel l'utilisateur n'exercerait pas d'inhalation entre l'ouverture et la fermeture. L'ouverture du réservoir, son vidage, la distribution de la poudre dans les poumons de l'utilisateur ainsi que le déplacement de la bande de blisters pour amener un nouveau réservoir plein en face des moyens d'ouverture n'est donc possible que si l'utilisateur inhale.

Par ailleurs, après inhalation et donc déplacement des moyens de support mobiles 50 vers la position de distribution, la fermeture de l'élément de capot mobile 12 ramène l'organe de chargement 800 vers sa position de départ. Comme visible sur la figure 6, c'est à ce moment là que la projection 820 de l'organe de chargement 800 coopère avec la partie d'extension 520 des moyens de support mobiles 50 pour pousser lesdits moyens de support mobiles 50 et ainsi les faire pivoter vers leur position de non-distribution. Il y a donc avantageusement un retour des moyens de support mobiles 50 vers la position de non-distribution qui est mécaniquement liée à la fermeture de l'élément de capot mobile 12.

Comme expliqué précédemment, les figures 2 à 9 ne représentent qu'un seul élément de capot mobile 12, mais il est entendu qu'un second élément de capot mobile, avantageusement symétrique par rapport à celui représenté, pourrait être prévu autour du corps 10, comme cela apparaît sur la figure 1. Avantageusement, ces deux éléments de capot mobiles 11, 12 sont alors engrenés l'un dans l'autre pour garantir une ouverture et une fermeture symétrique desdits deux éléments de capot mobiles. Cet engrenage peut se faire à proximité de leur point de pivotement.

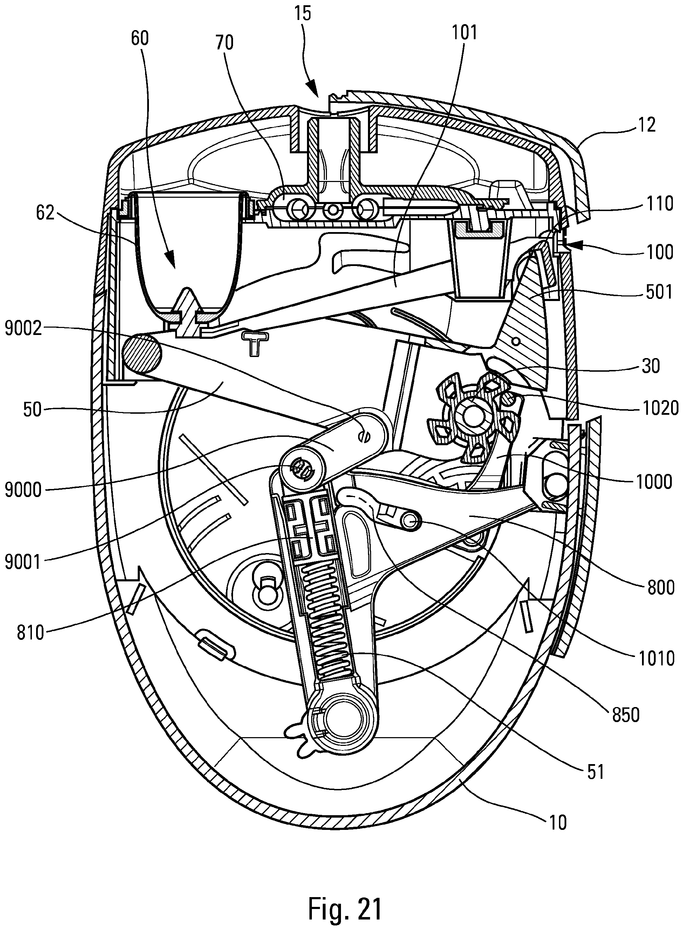

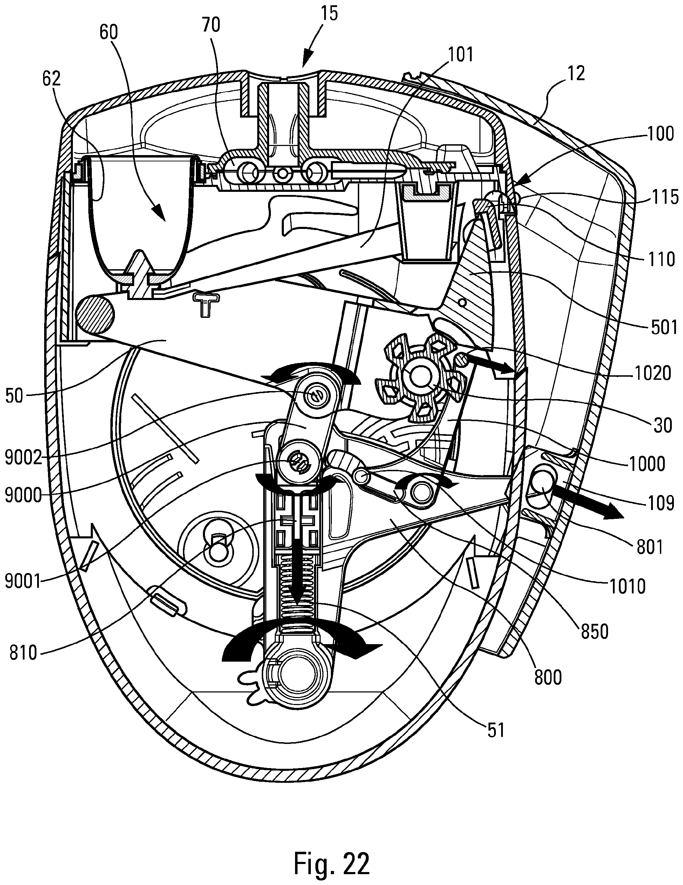

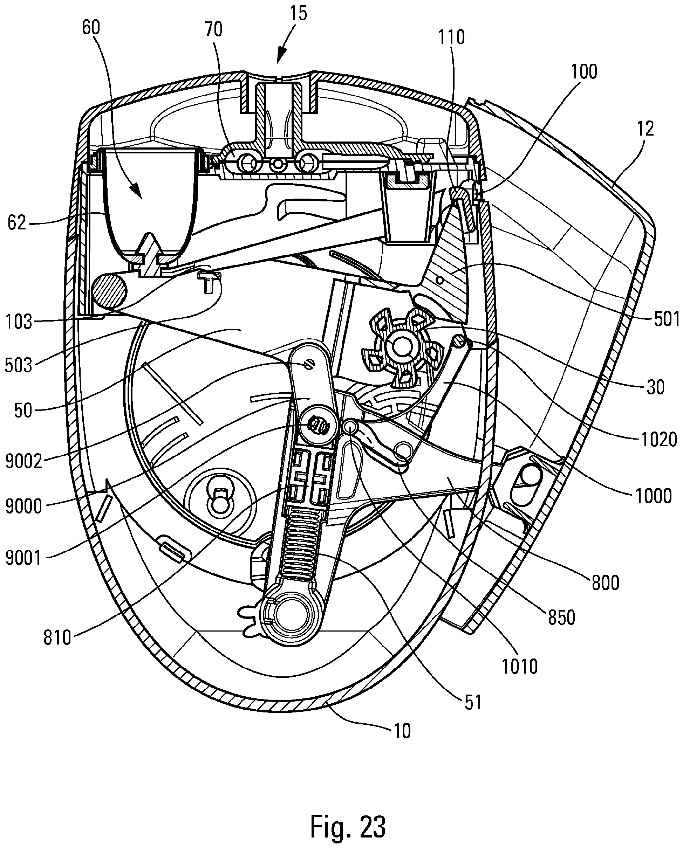

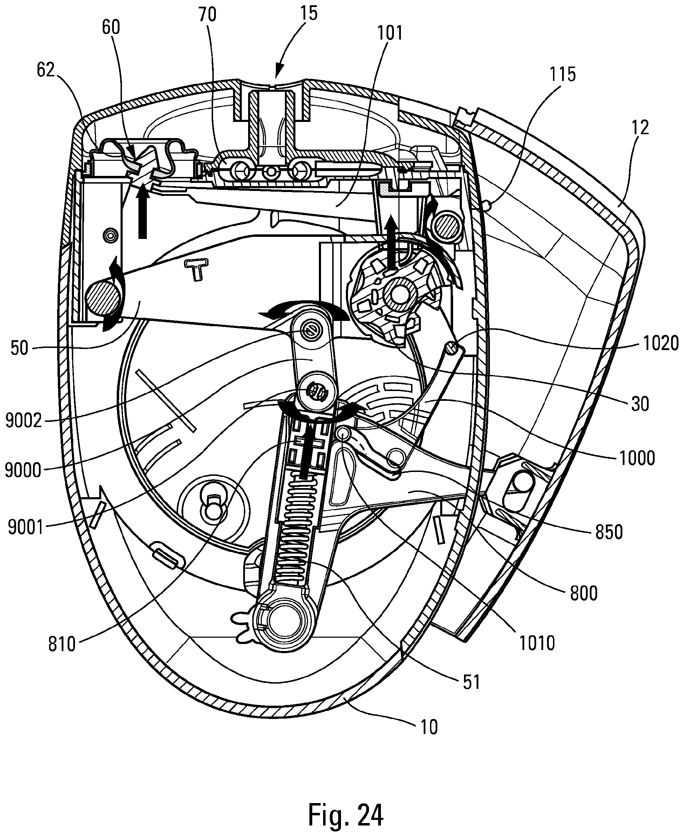

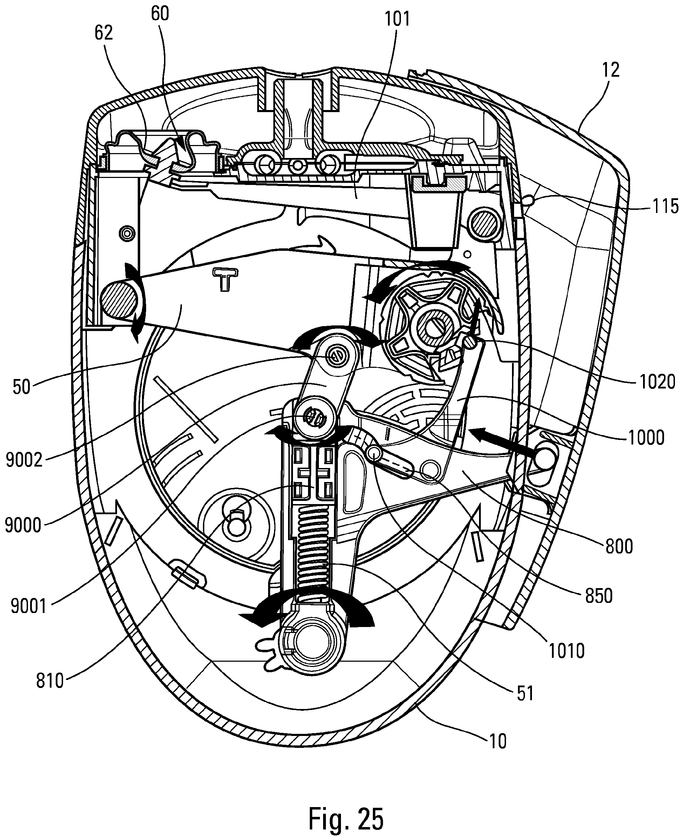

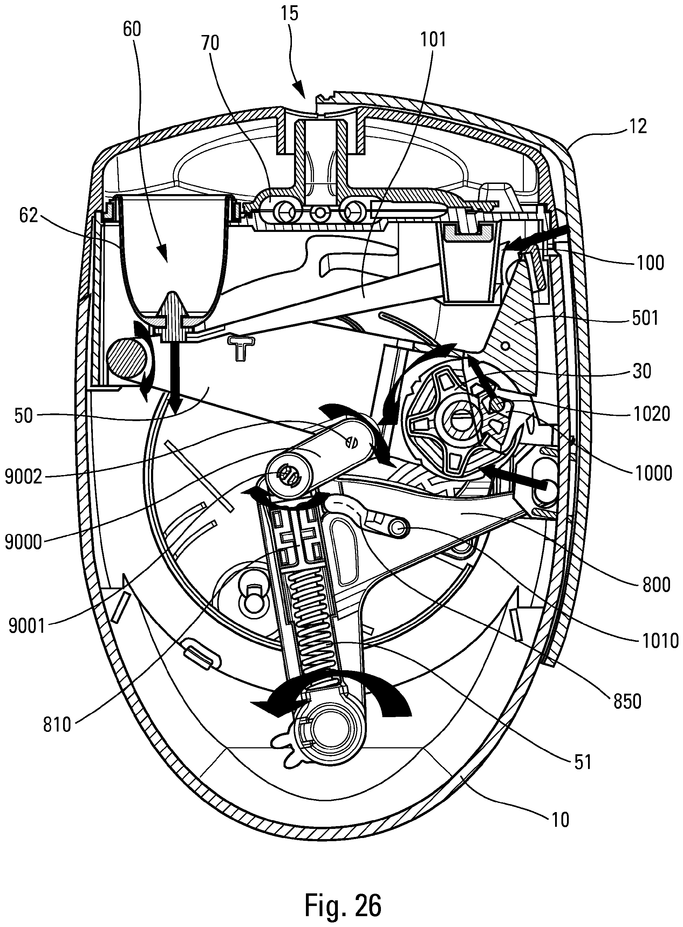

Les figures 21 à 26 montrent une variante de réalisation, dans laquelle la surface de came 910 est remplacée par une bielle ou genouillère 9000 connectée de manière pivotante d'une part à la tige 810 reliée au ressort 51, et d'autre aux moyens de support mobiles 50, par l'intermédiaire de deux axes de pivotement respectif 9001 et 9002. En position de fermeture, représentée sur la figure 21, la genouillère 9000 fait un angle par rapport à la tige 810 et à l'axe longitudinal du ressort 51. Lorsque l'utilisateur ouvre les éléments de capot 11, 12, il tire sur l'axe 9001 en déplaçant l'organe de chargement 800 vers la droite sur la figure, comme indiqué par les flèches sur la figure 22. Les moyens de support mobiles 50 étant fixes, car retenu par les moyens de blocage 100, la genouillère se redresse donc pour s'aligner avec l'axe de la tige 810 et du ressort 51, comme visible sur la figure 22. Ceci provoque la compression du ressort 51 du fait de la forme de la genouillère, plus longue que large. En position d'ouverture, visible sur la figure 23, la genouillère a avantageusement dépassé l'axe longitudinal de la tige 810 et du ressort 51, pour assurer une position d'ouverture stable. Comme précédemment, si l'utilisateur referme l'inhalateur sans inhaler, il ne se passe rien au niveau de la bande de blister, qui n'a pas bougé. Après inhalation (figure 24), les moyens de blocage se libèrent, comme décrit précédemment, un réservoir est ouvert et une dose est expulsée. Ensuite, lorsque l'utilisateur referme les éléments de capot 11,12 (figures 25 et 26), la genouillère 9000 pivote en retour vers sa position initiale, ce qui ramène les moyens de support mobiles 50 dans leur position de non distribution. Les moyens de blocage 100 et les moyens de solidarisation 1000 sont sensiblement identiques à ceux du mode de réalisation des figures 2 à 9, mais des variantes sont aussi envisageables.

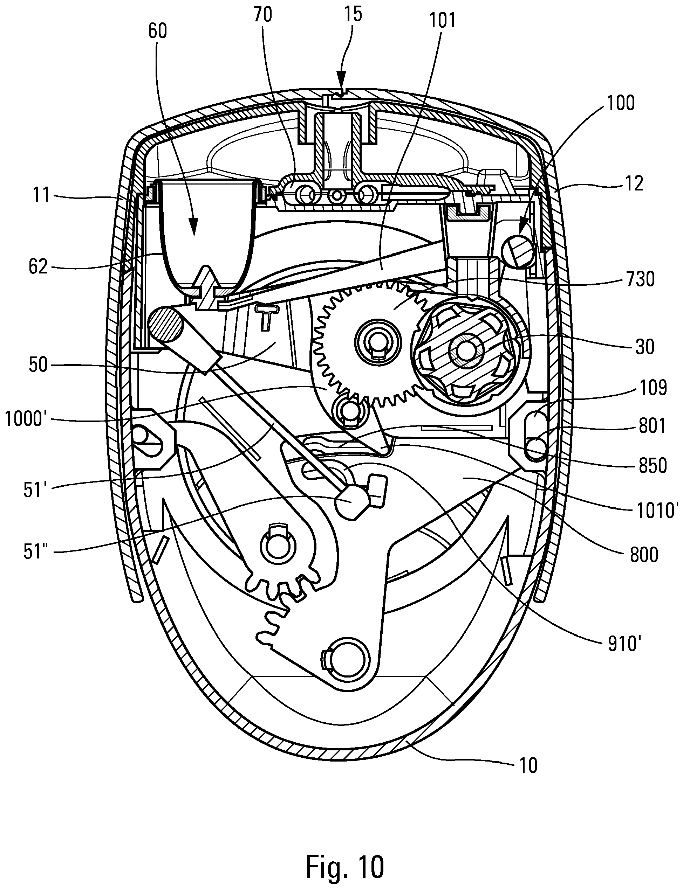

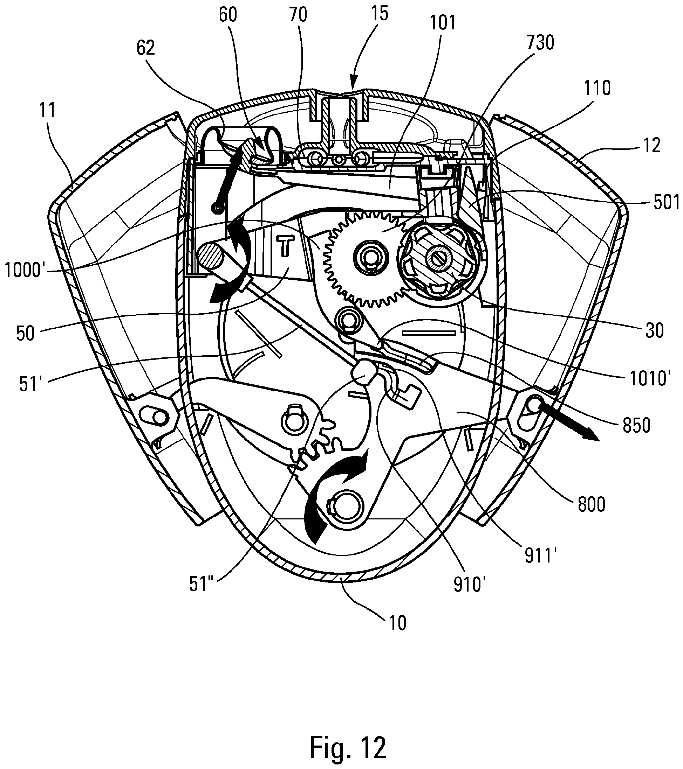

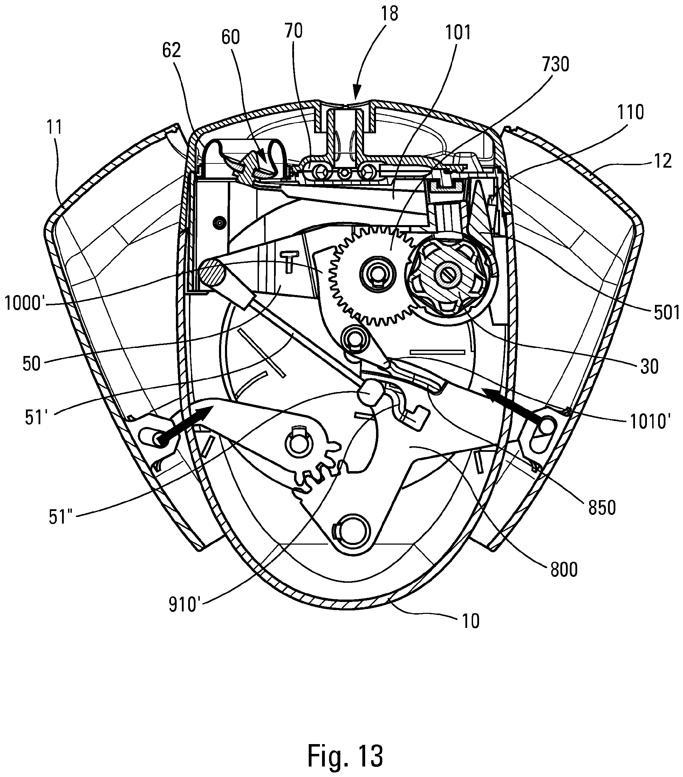

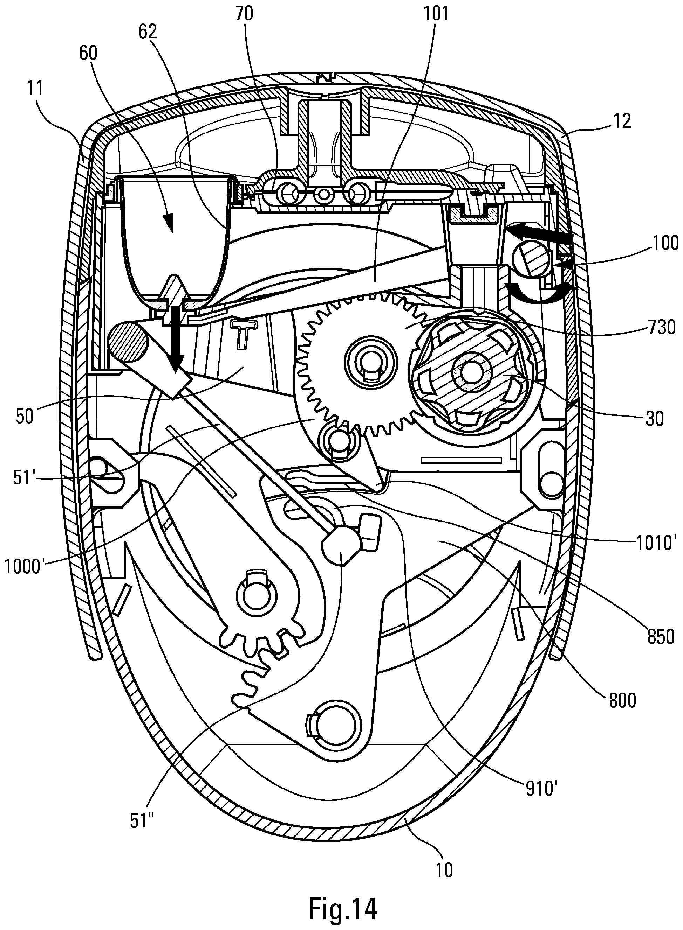

Les figures 10 à 14 représentent un autre mode de réalisation de l'invention. Dans ce mode de réalisation, les éléments identiques ou similaires seront représentés par des références numériques identiques, alors que les éléments qui diffèrent sont représentés par des références numériques comportant des primes.

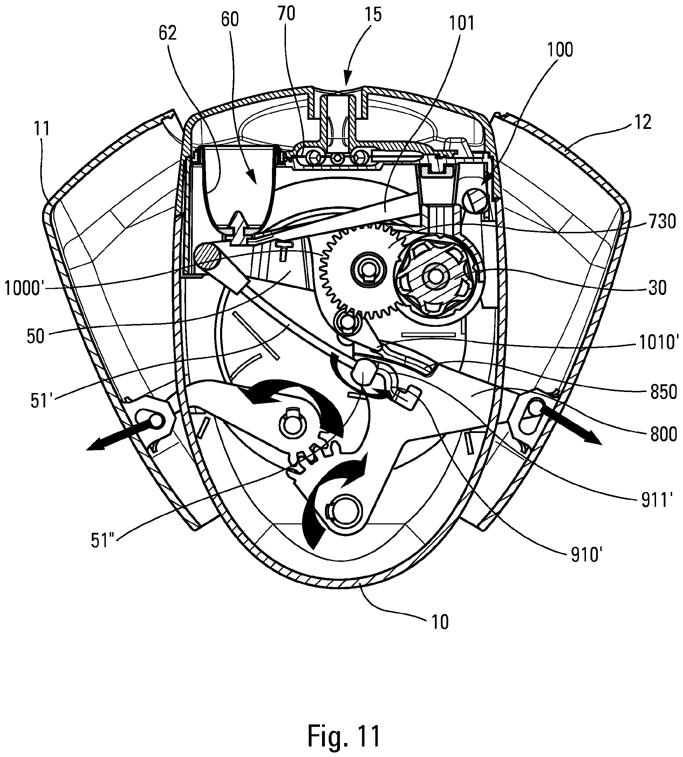

Ce mode de réalisation diffère principalement des précédents par les moyens de chargement qui sont réalisés différemment. Dans ce second mode de réalisation, il n'y a plus de ressort 51 mais une tige 51' qui peut se fléchir pour exercer la force élastique sur les moyens de support mobiles 50. Cette tige 51' est donc d'une part fixée sur les moyens de support mobiles 50, et d'autre part elle est connectée à l'organe d'armement 800, avantageusement par l'intermédiaire d'une projection 51" pénétrant dans une rainure 910' de forme appropriée. Cette rainure forme une surface de came 910' contre laquelle vient coulisser ladite projection 51" de la tige 51' lors de l'ouverture et de la fermeture des éléments de capot mobiles 11, 12. La forme de cette rainure, environ en forme d'arc de cercle ou de partie arquée, comporte ainsi une première partie de rainure et une seconde partie de rainure reliées au niveau d'un sommet 911'. Comme pour la surface de came 910 du premier mode de réalisation des figures 2 à 9, la première partie de la rainure 910' sert à charger le ressort, c'est-à-dire la tige élastique 51', en la déformant, alors que la seconde partie de la rainure 910' permet d'assurer une position stable en position chargée, c'est-à-dire en position d'ouverture. Lorsque le dispositif est chargé comme représenté sur la figure 11, la tige flexible fléchie 51' exerce donc une force sur les moyens de support mobiles 50 pour les solliciter en direction de leur position de distribution. De manière similaire au premier mode de réalisation des figures 2 à 9, ces moyens de support mobiles 50 sont maintenus en position de non distribution par les moyens de blocage 100, qui peuvent être réalisés de manière très similaire à celle décrite précédemment. Lorsque l'utilisateur inhale, les moyens de blocage 100 sont libérés et les moyens de support mobiles 50 peuvent se déplacer vers leur position de distribution en direction des moyens d'ouverture.

Une autre différence de ce mode de réalisation concerne les moyens de déplacement du support de réservoir, en particulier la bande de blisters. En effet, la roue de guidage 30 est, dans ce second mode de réalisation, engrenée avec une roue dentée 730, elle-même en coopération avec un élément d'entraînement 1000'. Alors que dans le premier mode de réalisation des figures 2 à 9, l'élément d'entraînement 1000 était relié d'une part à la rainure 850 de l'organe de chargement 800 et d'autre part directement à la denture 37 de la roue de guidage 30, ici l'élément d'entraînement 1000' est relié d'une part à la rainure 850 de l'organe de chargement 800, comme dans le premier mode de réalisation, mais d'autre part il est connecté à la roue dentée 730 interposée entre l'élément d'entraînement 1000' et la roue de guidage 30. Pour le reste, le fonctionnement est similaire au premier mode de réalisation, à savoir que lorsque l'utilisateur ouvre les éléments de capot mobiles 11,12, l'élément d'entraînement 1000' coulisse dans la rainure 850, avantageusement au niveau d'une projection 1010'. La première partie de rainure 850 étant sensiblement parallèle ou équidistante par rapport à l'axe de pivotement des éléments de capot mobiles 11,12, cette première partie de rainure ne provoque sensiblement pas d'action sur l'élément d'entraînement 1000', alors que la seconde partie de rainure, de pente différente, provoque un pivotement dudit élément d'entraînement 1000' qui se désengrène de la roue dentée 730. Si l'utilisateur referme le dispositif sans inhaler, l'élément d'entraînement 1000' coulisse simplement en retour dans la rainure 850 et revient s'engrener dans la même dent de la roue dentée 730, de sorte qu'il ne se passe rien pour le support de réservoir ou pour les moyens de blocage 100. Par contre, après inhalation, lorsque l'utilisateur referme les éléments de capot mobiles, l'élément d'entraînement 1000' vient s'engrener dans une autre dent de la roue dentée, de manière similaire à ce qui a été décrit précédemment avec le premier mode de réalisation.

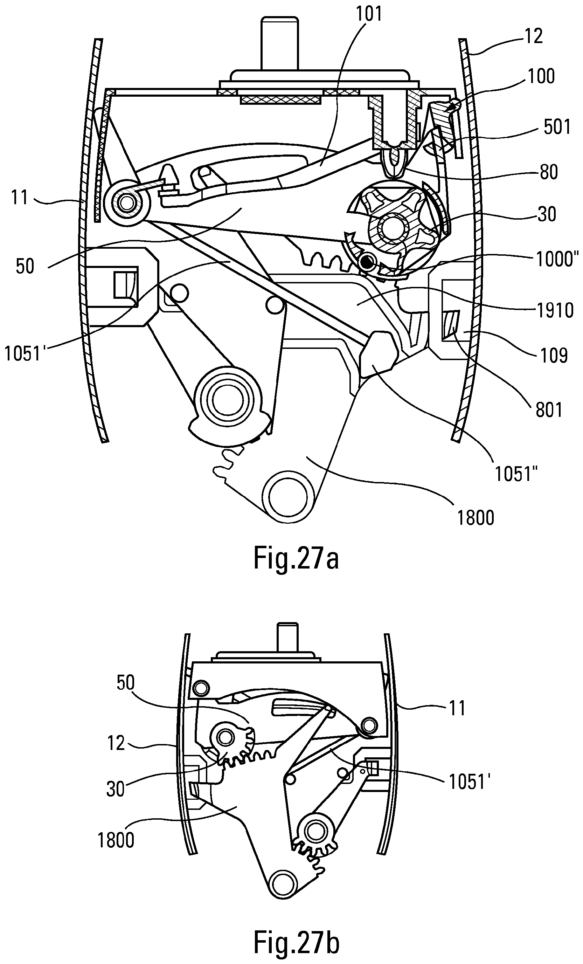

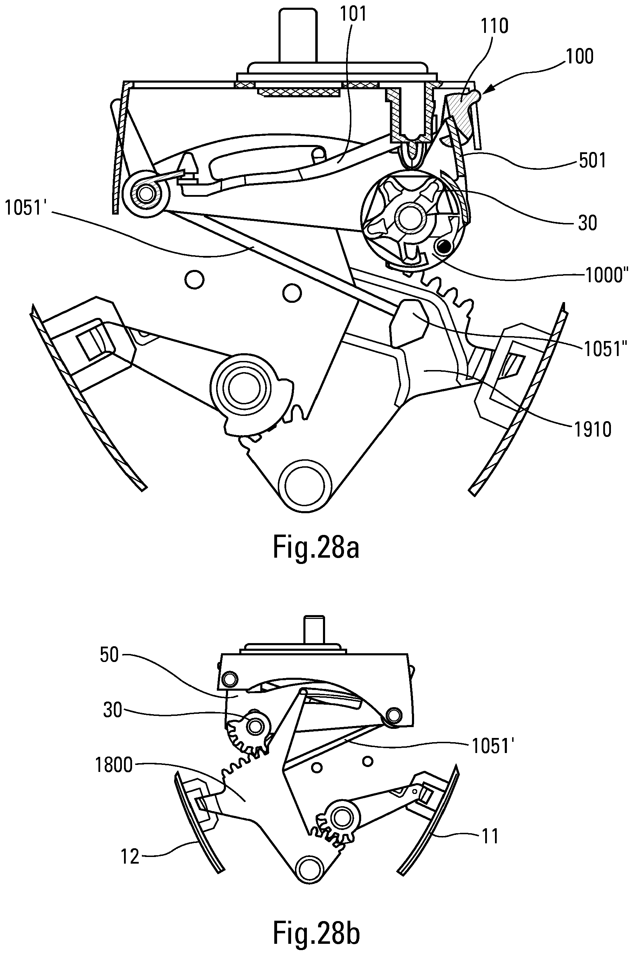

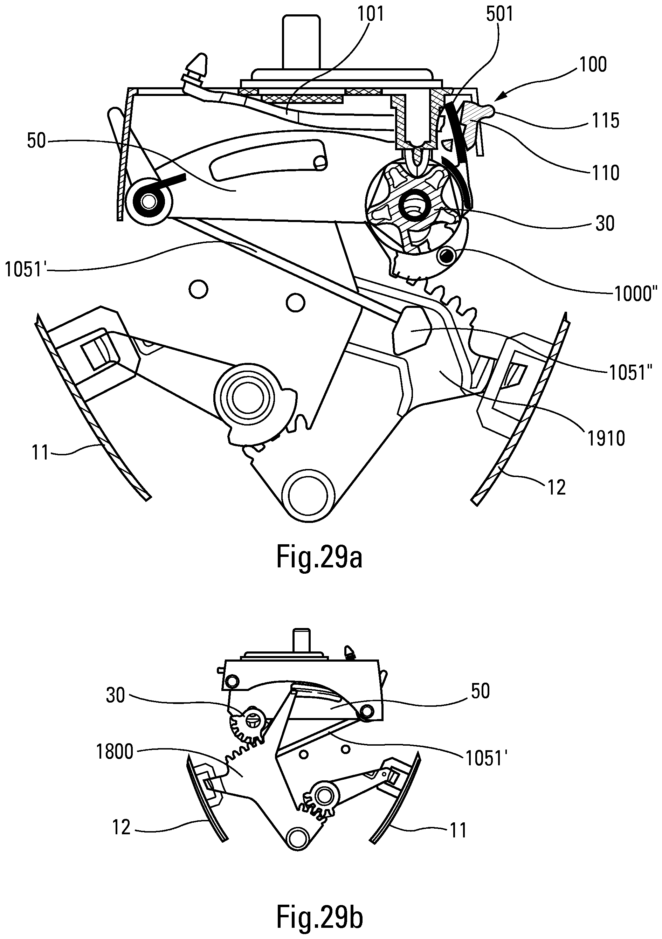

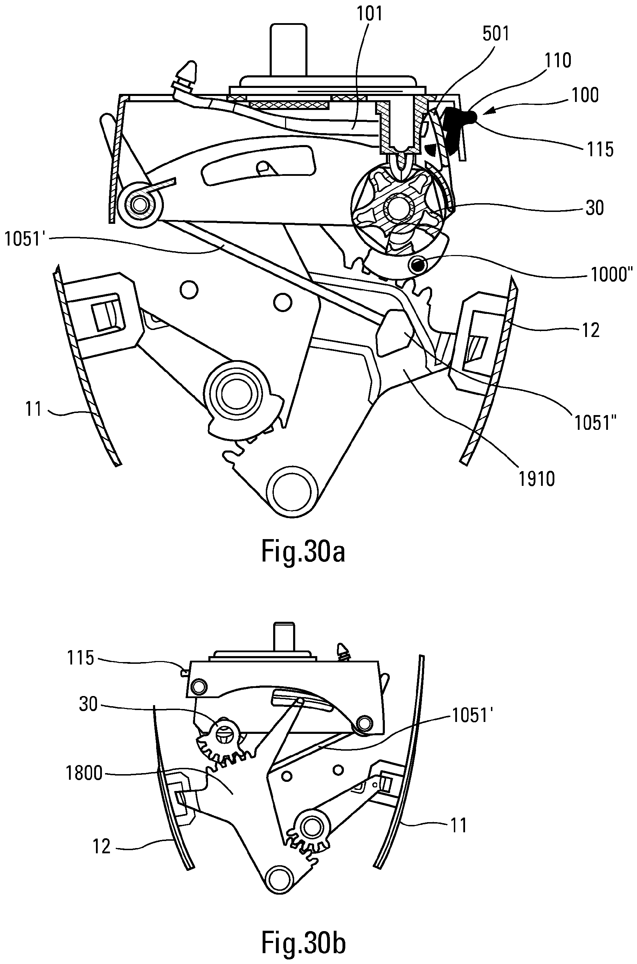

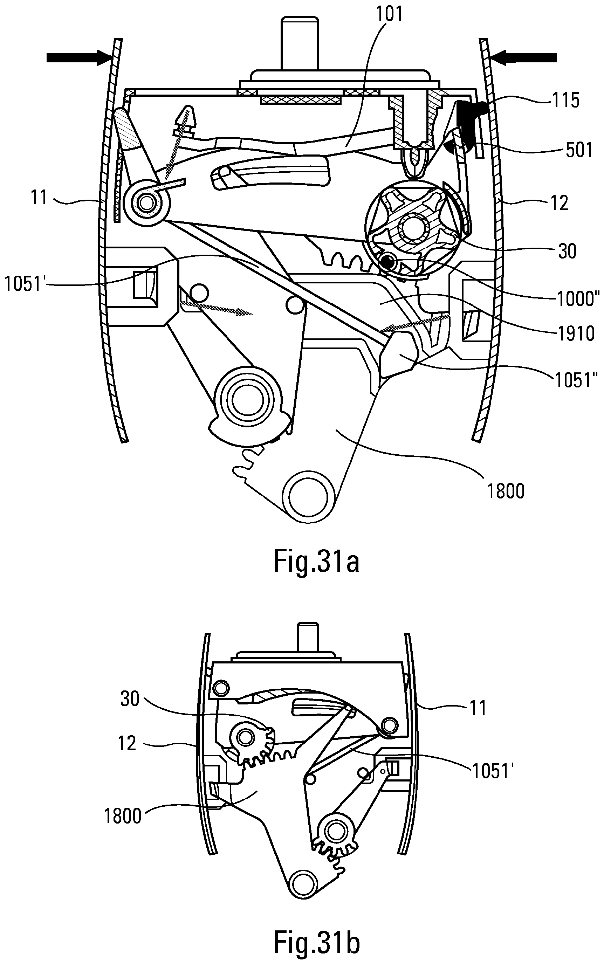

Les figures 27a à 31b montrent un autre mode de réalisation, dans lequel le support de réservoir 20 est déplacé dans la première direction à chaque ouverture des éléments de capot 11, 12. Dans cette variante, si l'utilisateur referme sans inhaler, il ramène le support de réservoir en retour vers sa position initiale. En l'absence d'inhalation, le support de réservoir effectue donc un mouvement de va-et-vient pour se retrouver exactement à sa position de départ après la fermeture. Ceci garantit donc également de ne pas perdre de dose, même en cas de manipulation incomplète du dispositif. En cas d'inhalation, la fermeture après inhalation ne provoque pas de déplacement du support de réservoir, de sorte que pour le prochain actionnement, ce sera le prochain réservoir plein qui sera amené en face des moyens d'ouverture lors de l'ouverture des éléments de capot.

Dans ce mode de réalisation, les moyens de chargement comprennent une tige 1051' fixée d'une part aux moyens de support mobiles 50, et d'autre part coulissant par l'intermédiaire d'une projection 1051" dans une rainure 1910 prévue dans un organe de chargement 1800, relié auxdits éléments de capot mobiles 11, 12. Le fonctionnement de l'armement est similaire à celui décrit en référence aux figures 10 à 14. L'organe de chargement 1800 est engrené avec la roue de guidage 30, comme cela est mieux visible sur les vues de derrière. Des moyens de solidarisation 1000" sont prévus pour coopérer avec la roue de guidage 30 tant que les moyens de support mobiles 50 n'ont pas été déplacés dans leur position de distribution, après une inhalation. Après inhalation, ces moyens de solidarisation, comportant avantageusement une projection coopérant avec une denture de la roue de guidage 30, sont désactivés, c'est-à-dire qu'ils ne coopèrent plus avec la roue de guidage. Lorsque l'utilisateur referme le dispositif, la roue de guidage revient donc ensemble avec les moyens de support mobiles vers la position de non distribution, sans tourner autour de son axe de rotation. Ce n'est qu'en fin de fermeture que les moyens de solidarisation s'engrènent à nouveau avec la roue de guidage.

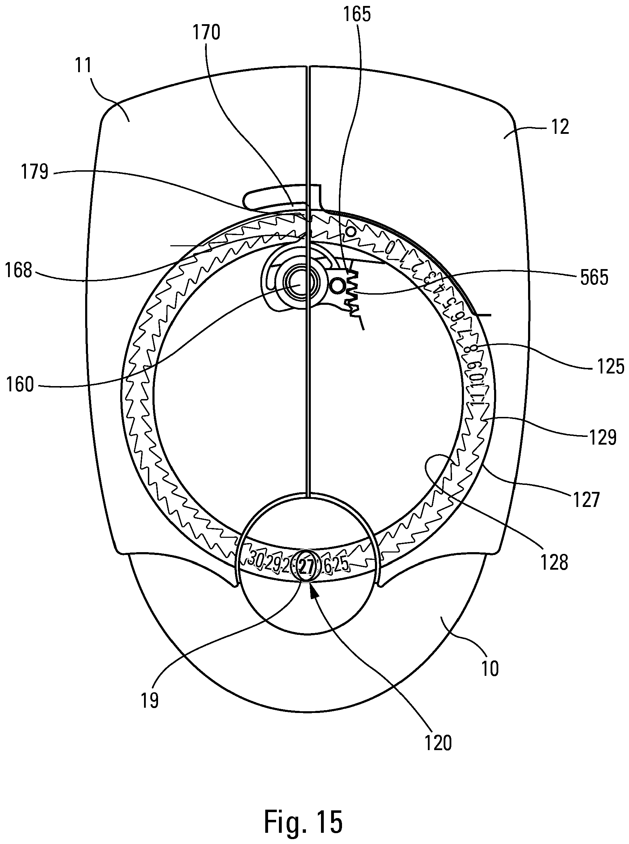

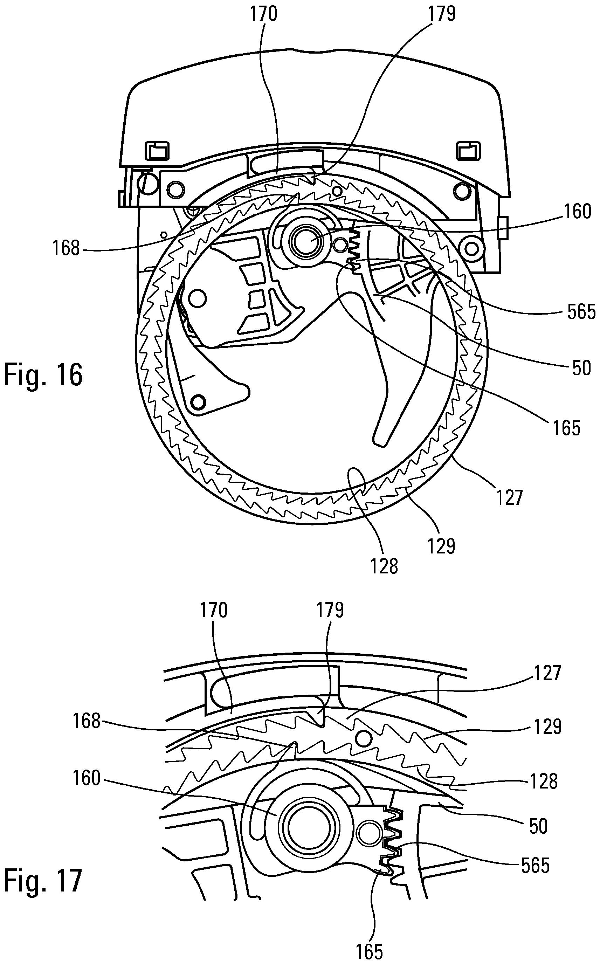

Le dispositif de l'invention peut également comporter une indicateur ou compteur de doses 120, adapté à compter ou à indiquer à l'utilisateur le nombre de doses distribuées ou restant à distribuer. Dans l'exemple représenté, l'indicateur est adapté à compter 60 doses. Les figures 15 à 20 montrent un cycle d'actionnement du dispositif et la manière dans laquelle l'indicateur est actionné. Cet indicateur comporte avantageusement une bague 127 pourvue d'une denture interne 128 et d'une denture externe 129 et comportant des chiffres 125, par exemple de 0 à 60, imprimés sur une de ses faces. La bague est montée de telle sorte que les chiffres passent successivement dans la fenêtre 19 du corps 10. La denture interne 128 est avantageusement adaptée à coopérer avec un actionneur 160, alors que la denture externe 129 est avantageusement adaptée à coopérer avec des moyens anti-retour 170, qui sont adaptés à éviter une rotation de la bague d'indication 127 en sens inverse de celui qui lui est imparti par l'actionneur 160.

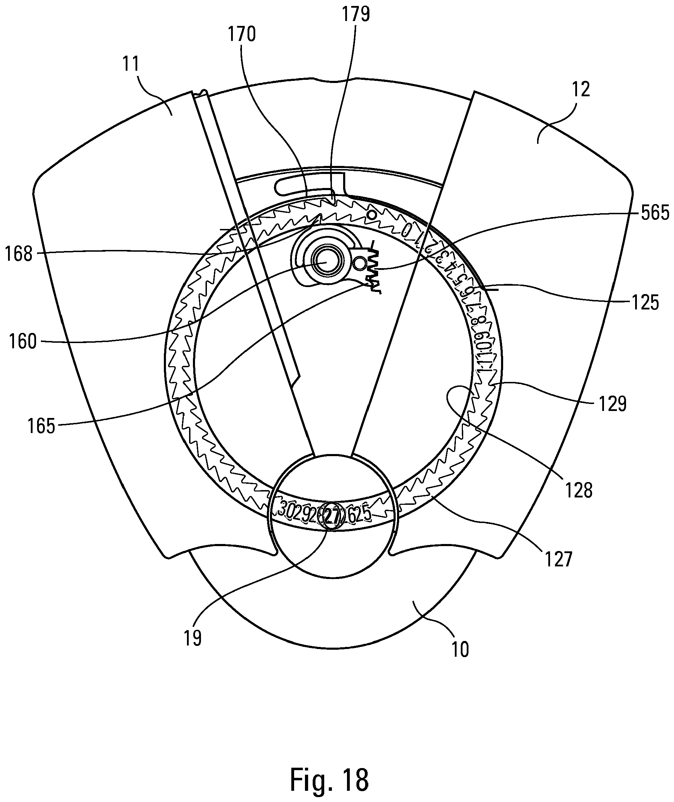

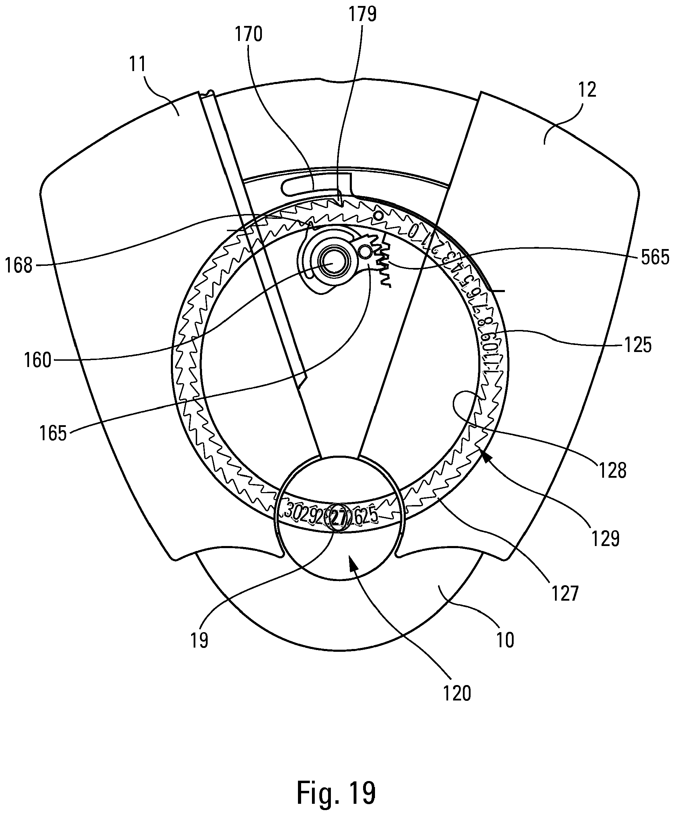

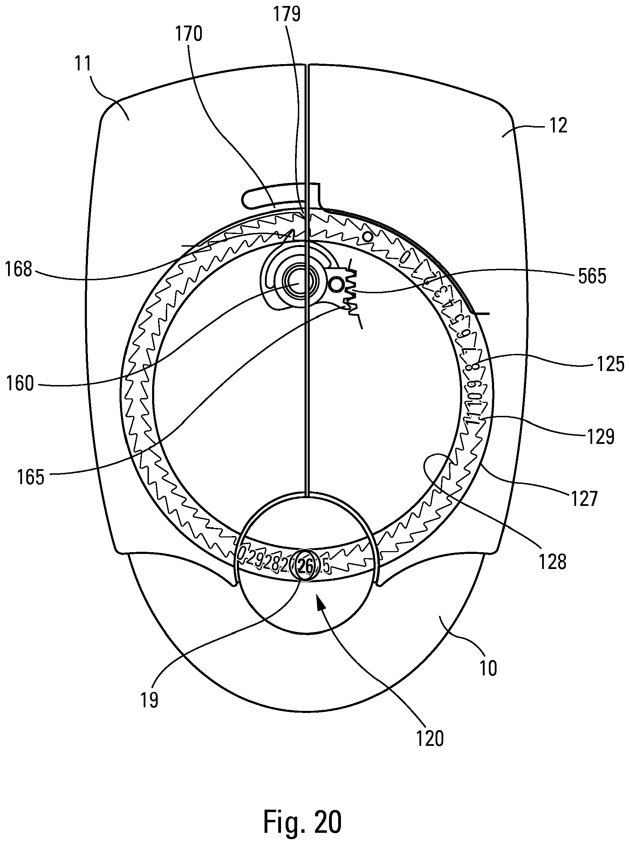

Un but de l'invention est d'éviter de compter des doses qui ne sont pas distribuées, par exemple en cas d'erreur de manipulation, ou d'une manipulation incomplète du dispositif. Il est donc essentiel que le compteur ou indicateur ne soir actionné qu'une fois que l'utilisateur a inhalé, puisque c'est cette inhalation qui conditionne l'ouverture et la distribution de la dose de chaque blister. Pour ce faire, le dispositif comporte un actionneur 160 monté pivotant sur le corps 10. Cet actionneur 160 comporte des moyens de connexion 165, notamment des dents, adaptés à s'engrener dans une denture 565 ou des dents complémentaires prévues sur des moyens de support mobiles 50. Ainsi, lorsque l'utilisateur ouvre le dispositif et qu'il charge les moyens de chargement du dispositif, les moyens de support mobiles 50 ne bougent pas puisqu'ils sont maintenus en position de non-distribution par les moyens de blocage 100. Il ne se passe donc rien au niveau de l'indicateur puisque l'actionneur 160, monté pivotant sur le corps 10 et engrené avec les moyens de support mobiles 50, reste également immobile. Si l'utilisateur referme le dispositif sans inhaler, il ne se passe évidemment rien non plus puisque les moyens de support mobiles 50 restent toujours immobiles. De cette manière, on garantit que l'indicateur ne compte pas de dose s'il n'y a pas d'inhalation. A partir de la position chargée, représentée sur la figure 18, si l'utilisateur inhale, il provoque le déplacement des moyens de support mobiles 50 dans leur position de distribution en direction des moyens d'ouverture. Ce déplacement provoque donc le pivotement de l'actionneur 160 dans une première direction comme visible sur les figures 18 et 19. L'actionneur 160 comporte un doigt 168 engrené dans la denture interne 128 de la bague d'indication 127. Dans cette première direction de déplacement, le doigt 168 de l'actionneur peut glisser sur la pente inclinée de la dent correspondante pour venir se positionner face à la prochaine dent. Parallèlement, les moyens anti-retour 170, en particulier un doigt anti-retour 179, coopèrent avec la denture externe 129 de la bague 127 pour empêcher une rotation de celle-ci sous l'effet du frottement, exercé par exemple par le doigt 168 de l'actionneur sur la denture interne 128. Après l'inhalation, lorsque l'utilisateur referme le dispositif, les moyens de support mobiles 50 sont ramenés vers leur position de repos, c'est-à-dire de non-distribution. Ce mouvement provoque donc un pivotement de l'actionneur 160 en sens inverse du précédent, puisque les engrenages respectifs 165, 565 de l'actionneur et des moyens de support mobiles pivotent en sens inverse à celui décrit précédemment. Dans ce déplacement en sens inverse, le doigt 168 de l'actionneur 160 pousse à l'intérieur de la dent dans laquelle il est positionné pour faire tourner la bague 127, comme visible sur la figure 20. Parallèlement, le doigt anti-retour 179 glisse sur la pente inclinée de la dent pour venir se positionner dans la dent suivante. Dans l'exemple représenté, l'indicateur est adapté à indiquer le nombre de doses restant à distribuer de sorte que le chiffre affiché diminue à chaque actionnement. L'inverse est bien entendu également possible, à savoir un compteur qui compte le nombre de doses distribuées. Avantageusement, on peut prévoir des moyens de blocage de l'indicateur après distribution de la dernière dose. Ces moyens de blocage peuvent prendre différentes formes, une forme avantageuse étant de prévoir une dent de forme différente sur la denture interne de sorte que l'actionneur ne peut plus venir s'engrener dans la dent suivante pour continuer à faire tourner ladite bague d'indication. D'autres moyens pour bloquer la rotation de la bague après distribution de la dernière dose sont également envisageables.

Dans tous les modes de réalisation décrits ci-dessus, la bande de blister est formée par une bande présentant deux extrémités. En variante, on pourrait utiliser une bande continue. D'autres modifications sont également possibles sans sortir du cadre de la présente invention.

La présente invention permet donc de fournir un inhalateur de poudre sèche qui procure notamment les fonctions suivantes :
▪ une pluralité de doses individuelles de poudre stockées dans des réservoirs individuels étanches, par exemple 30 ou 60 doses stockées sur une bande enroulée en bobine ;
▪ la poudre libérée au moyen d'un perçage actionné par l'inhalation de l'utilisateur, ce perçage du blister étant réalisé au moyen d'un système de détection d'inhalation couplé à un système de libération préchargé ;
▪ des moyens d'entraînement de forme appropriée en prises avec les blisters pour réaliser le déplacement de la bande de blister à chaque actionnement, et amener un nouveau réservoir dans une position dans laquelle il est destiné à être ouvert par les moyens d'ouverture appropriés ;
▪ des moyens pour éviter une perte de dose en cas d'ouverture de l'inhalateur mais en l'absence d'inhalation. Dans ce cas, lors de la fermeture de l'inhalateur, le dispositif revient exactement à sa position de départ ;
▪ un indicateur de doses adapté à compter les doses seulement en cas d'inhalation.

D'autres fonctions sont également fournies par le dispositif de l'invention tel que cela a été décrit précédemment. Il est à noter que les différentes fonctions, même si elles ont été représentées comme prévues simultanément sur les différents modes de réalisation de l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les réservoirs individuels sont arrangés les uns par rapport aux autres, etc. Les moyens d'armement et le système de déclenchement par l'inhalation pourraient être réalisés différemment. Il en est de même des autres parties constitutives du dispositif.

L'inhalateur de l'invention, incorporant toutes ou parties des fonctions décrites, s'avère fournir des performances supérieures à celles des dispositifs existants. En particulier, l'inhalateur de l'invention fournit de préférence un taux de vidage des réservoirs d'au moins 90% à chaque actionnement. Avantageusement, ce taux de vidage, correspondant au pourcentage du produit fluide expulsé hors d'un réservoir ouvert lors d'un actionnement du dispositif, est supérieur à 95%, de préférence même supérieur à 97%. Ce taux de vidage élevé est notamment même supérieur aux performances obtenues avec des inhalateurs actifs qui sont généralement plus efficaces que les inhalateurs passifs, et dans lesquels ce n'est pas l'écoulement d'inhalation qui vide le blister et expulse la dose mais un écoulement d'air comprimé libéré au moment de l'inhalation. Il garantit une efficacité maximale du dispositif de l'invention. Couplé à l'ouverture déclenchée par l'inhalation, ce taux de vidage élevé garantit une distribution optimale du produit fluide, en l'occurrence la poudre, dans les poumons de l'utilisateur. Par ailleurs, l'invention fournit également une meilleure régularité de vidage des réservoirs lors d'actionnements successifs. Ainsi, en se référant par exemple à 10 réservoirs d'une bande de blisters, il s'avère que le taux de vidage varie de moins de 15%, avantageusement de moins de 10%, de préférence de moins de 5% d'un réservoir à l'autre. Cette meilleure régularité garantit une meilleure reproductibilité de la dose et donc aussi une meilleure efficacité du dispositif de l'invention.

Diverses modifications sont également possibles pour un homme du métier sans sortir du cadre de la présente invention tel que défini par les revendications annexées. En particulier, les diverses caractéristiques et fonctionnalités du dispositif décrites en référence aux différents modes de réalisation et variantes peuvent être adaptées à tous ces modes de réalisation et variantes, et peuvent être combinées entre elles d'une quelconque façon appropriée.

## Revendications

1. Dispositif de distribution de produit fluide comportant :
- un corps (10) pourvu d'un orifice de distribution,
- au moins un élément de capot (11, 12) mobile entre une position de fermeture et une position d'ouverture,
- une pluralité de réservoirs individuels (21) contenant chacun une dose de produit fluide, telle que de la poudre pharmaceutique, lesdits réservoirs étant formés sur un support de réservoirs (20),
- des moyens de support mobiles (50) recevant ledit support de réservoir et déplaçables entre une position de non-distribution et une position de distribution,
- des moyens d'ouverture de réservoir (80) pour ouvrir un réservoir respectif à chaque actionnement desdits moyens d'ouverture,
- ledit support de réservoir étant déplaçable ensemble avec lesdits moyens de support mobiles, entre la position de non-distribution dans laquelle il ne coopère pas avec lesdits moyens d'ouverture, et la position de distribution, dans laquelle lesdits moyens d'ouverture ouvrent un réservoir respectif,
- des moyens de chargement (800) pour solliciter lesdits moyens de support mobiles vers ladite position de distribution,
- des moyens de blocage (100) pour maintenir lesdits moyens de support mobiles en position de non-distribution,
- des moyens de déclenchement (60), pour libérer lesdits moyens de blocage et permettre le déplacement desdits moyens de support mobiles, ensemble avec ledit support de réservoir, vers ladite position de distribution,
- lesdits moyens de chargement comprenant un élément de chargement élastiquement déformable (51 ;51' ; 1051') coopérant avec une surface de came (910 ; 910' ; 1910),
**caractérisé en ce que** ladite surface de came (910 ; 910' ; 1910) comporte au moins deux parties de pente différentes, une première partie de surface de came adaptée à déformer et/ou charger lesdits moyens de chargement, et une seconde partie de surface de came coopérant avec lesdits moyens de chargement déformés et/ou chargés en position d'ouverture du dispositif.

2. Dispositif selon la revendication 1, dans lequel ledit élément de chargement élastiquement déformable comprend un ressort comprimable (51).

3. Dispositif selon la revendication 2, dans lequel une tige (810) est interposée entre ledit ressort (51) et ladite surface de came (910), le déplacement de la tige (810) sur ladite surface de came (910) comprimant et/ou décomprimant ledit ressort (51).

4. Dispositif selon la revendication 3, dans lequel ladite surface de came (910) est réalisée sur lesdits moyens de support mobiles (50) et présente une première partie inclinée et une seconde partie de pente inférieure, ladite tige (810) coopérant avec ladite seconde partie de surface de came lorsque le ressort (51) est comprimé, de telle sorte que la force exercée par ladite tige (810) sur ladite seconde partie de surface de came est sensiblement perpendiculaire à ladite seconde partie de surface de came.

5. Dispositif selon la revendication 3 ou 4, dans lequel l'extrémité (811) de la tige (810) en contact avec la surface de came (910) présente un profil facilitant le glissement sur ladite surface de came.

6. Dispositif selon la revendication 5, dans lequel ledit profil comporte une surface au moins partiellement arrondie, de préférence sphérique.

7. Dispositif selon la revendication 1, dans lequel ledit élément de chargement élastiquement déformable comprend une tige fléchissable (51' ; 1051').

8. Dispositif selon la revendication 7, dans lequel ladite tige (51' ; 1051') est fixée sur lesdits moyens de support mobiles (50) et comporte une projection (51" ; 1051") coopérant avec ladite surface de came (910' ; 1910).

9. Dispositif selon la revendication 8, dans lequel ladite surface de came (910' ; 1910) est formée par une rainure réalisée dans lesdits moyens de chargement (800) et dans laquelle coulisse ladite projection (51" ; 1051").

10. Dispositif selon la revendication 9, dans lequel ladite rainure (910' ; 1910) comporte une première partie de rainure pour fléchir ladite tige (51' ; 1051') et une seconde partie de rainure, ladite tige (51' ; 1051') coopérant avec ladite seconde partie de rainure à l'état fléchi.

11. Dispositif selon l'une quelconque des revendications précédentes, dans lequel lesdits moyens de blocage (100) comportent une tige (101) reliée d'un côté à des moyens déformables sous l'effet de l'inhalation (60), et comportant de l'autre côté un élément de blocage (110) adapté à coopérer avec lesdits moyens de support mobiles (50).

12. Dispositif selon la revendication 11, dans lequel, en position d'ouverture dudit au moins un élément de capot mobile (11, 12), lesdits moyens de support mobiles (50) exercent une force sur ledit élément de blocage (110).

13. Dispositif selon la revendication 12, dans lequel ladite tige (101) des moyens de blocage (100) comporte une zone d'appui (103) adaptée à coopérer avec une zone complémentaire (503) sur lesdits moyens de support mobiles (50), de telle sorte qu'en position d'ouverture, ladite zone d'appui (103) de la tige (101) exerce une force sur lesdits moyens de support mobiles (50), ladite force étant de direction sensiblement opposée à la force exercée par lesdits moyens de support mobiles (50) sur ledit élément de blocage (110), pour assurer la stabilité de la position d'ouverture.

14. Dispositif selon la revendication 13, dans lequel ladite zone d'appui (103) est à proximité du côté relié aux moyens déformables par l'inhalation (60).

15. Dispositif selon la revendication 13 ou 14, dans lequel la force exercée par lesdits moyens de support mobiles (50) sur l'élément de blocage (110) est supérieure à la force exercée par la zone d'appui (103) de la tige (101) sur lesdits moyens de support mobiles (50).

## Patentansprüche

1. Spender für ein Fluidprodukt, umfassend:
- Einen Körper (10), der mit einer Abgabeöffnung versehen ist,
- mindestens ein Abdeckelement (11,12), das aus einer geschlossenen Stellung in eine geöffnete Stellung und aus einer geöffneten Stellung in eine geschlossene Stellung bewegbar ist;
- eine Vielzahl von Einzelbehältern (21), von denen jeder eine Dosis des Fluidprodukts enthält, beispielsweise pharmazeutisches Pulver, wobei die Einzelbehälter auf einem Behälterträger (20) ausgebildet sind,
- bewegliche Halterungen (50), die den Behälterträger aufnehmen und zwischen einer Nichtabgabestellung und einer Abgabestellung verschiebbar sind,
- Behälteröffnungsmittel (80) zum Öffnen eines jeweiligen Behälters bei jeder Betätigung der Öffnungsmittel,
- wobei der Behälterträger zusammen mit den beweglichen Halterungen verschiebbar ist zwischen der Nichtabgabestellung, in der er nicht mit den Öffnungsmitteln zusammenwirkt, und der Abgabestellung, in der die Öffnungsmittel einen jeweiligen Behälter öffnen,
- Lademittel (800), um die beweglichen Halterungen in Richtung der Abgabestellung vorzuspannen,
- Blockiermittel (100), um die beweglichen Halterungen in Nichtabgabestellung zu halten,
- Auslösemittel (60), um die Blockiermittel zu lösen und die Verschiebung der beweglichen Halterungen, zusammen mit dem Behälterträger, zur Abgabestellung hin zu ermöglichen,
- wobei die Lademittel ein elastisch verformbares Ladeelement (51; 51'; 1051') umfassen, welches mit einer Nockenfläche (910; 910'; 1910) zusammenwirkt
**dadurch gekennzeichnet, dass** diese Nockenfläche (910; 910'; 1910) mindestens zwei unterschiedliche Steigungsabschnitte umfasst, wobei ein erster Abschnitt der Nockenfläche derart ausgelegt ist, dass er die Lademittel verformt und/oder lädt, und ein zweiter Abschnitt der Nockenfläche mit den verformten und/oder geladenen Lademitteln in der geöffneten Stellung der Vorrichtung zusammenwirkt.

2. Vorrichtung nach Anspruch 1, bei der das elastisch verformbare Ladeelement eine zusammendrückbare Feder (51) umfasst.

3. Vorrichtung nach Anspruch 2, bei der ein Stift (810) zwischen der Feder (51) und der Nockenfläche (910) angeordnet ist, wobei bei Verschiebung des Stifts (810) an der Nockenfläche (910) die Feder (51) zusammengedrückt und/oder entspannt wird.

4. Vorrichtung nach Anspruch 3, bei der die Nockenfläche (910) an den beweglichen Halterungen (50) ausgeführt ist und einen ersten schrägen Abschnitt und einen zweiten Abschnitt geringerer Steigung aufweist, wobei der Stift (810) bei zusammengedrückter Feder (51) derart mit dem zweiten Abschnitt der Nockenfläche zusammenwirkt, dass die von dem Stift (810) auf den zweiten Abschnitt der Nockenfläche ausgeübte Kraft im Wesentlichen senkrecht zum zweiten Abschnitt der Nockenfläche verläuft.

5. Vorrichtung nach Anspruch 3 oder 4, bei der das Ende (811) des Stifts (810), welches mit der Nockenfläche (910) in Kontakt steht, mit einem Profil versehen ist, das das Gleiten auf der Nockenfläche erleichtert.

6. Vorrichtung nach Anspruch 5, bei der das Profil eine zumindest teilweise abgerundete, vorzugsweise kugelförmige, Oberfläche aufweist.

7. Vorrichtung nach Anspruch 1, bei der das elastisch verformbare Ladeelement einen biegbaren Stift (51'; 1051') umfasst.

8. Vorrichtung nach Anspruch 7, bei der der biegbare Stift (51'; 1051') an den beweglichen Halterungen (50) angebracht ist und einen Vorsprung (51"; 1051") umfasst, der mit der Nockenfläche (910'; 1910) zusammenwirkt.

9. Vorrichtung nach Anspruch 8, bei der die Nockenfläche (910'; 1910) durch eine Nut gebildet wird, welche in den Lademitteln (800) ausgebildet ist und in der der Vorsprung (51'; 1051") gleitet.

10. Vorrichtung nach Anspruch 9, bei der die Nut (910'; 1910) einen ersten Nutabschnitt zum Biegen des Stifts (51'; 1051') umfasst, und einen zweiten Nutabschnitt, wobei der Stift (51'; 1051') im gebogenen Zustand mit dem zweiten Nutabschnitt zusammenwirkt.

11. Vorrichtung nach einem der vorhergehenden Ansprüche, bei der die Blockiermittel (100) einen Stift (101) umfassen, der an der einen Seite mit Mitteln verbunden ist, die unter der Einwirkung der Einatmung (60) verformbar sind, und an der anderen Seite ein Blockierelement (110) umfasst, das zum Zusammenwirken mit den beweglichen Halterungen (50) ausgelegt ist.

12. Vorrichtung nach Anspruch 11, bei der die beweglichen Halterungen (50) in der geöffneten Stellung des mindestens einen beweglichen Abdeckelements (11, 12) eine Kraft auf das Blockierelement (110) ausüben.

13. Vorrichtung nach Anspruch 12, bei der der Stift (101) der Blockiermittel (100) einen Auflagebereich (103) umfasst, der zum Zusammenwirken mit einem komplementären Bereich (503) an den beweglichen Halterungen (50) ausgelegt ist, so dass, in der geöffneten Stellung, der Auflagebereich (103) des Stifts (101) eine Kraft auf die beweglichen Halterungen (50) ausübt, wobei diese Kraft in ihrer Richtung im Wesentlichen entgegengesetzt zu der von den beweglichen Halterungen (50) auf das Blockierelement (110) ausgeübten Kraft verläuft, um die Stabilität der geöffneten Stellung zu gewährleisten.

14. Vorrichtung nach Anspruch 13, bei der sich der Auflagebereich (103) in der Nähe der Seite befindet, die mit den durch die Einatmung (60) verformbaren Mitteln verbunden ist.

15. Vorrichtung nach Anspruch 13 oder 14, bei der die von den beweglichen Halterungen (50) auf das Blockierelement (110) ausgeübte Kraft größer ist als die Kraft, die der Auflagebereich (103) des Stifts (101) auf die beweglichen Halterungen (50) ausübt.

## Claims

1. A fluid dispenser device comprising:
• a body (10) provided with a dispenser orifice;
• at least one cover element (11, 12) that is movable between a closed position and an open position;
• a plurality of individual reservoirs (21) each containing a dose of fluid, such as a pharmaceutical powder, said reservoirs being formed on a reservoir substrate (20);
• movable support means (50) that receive said reservoir substrate, and that are displaceable between a non-dispensing position and a dispensing position;
• reservoir opening means (80) for opening a respective reservoir each time said opening means are actuated;
• said reservoir substrate being displaceable, together with said movable support means, between the non-dispensing position, in which it does not co-operate with said opening means, and the dispensing position, in which said opening means open a respective reservoir;
• loading means (800) for urging said movable support means towards said dispensing position;
• blocking means (100) for retaining said movable support means in the non-dispensing position;
• trigger means (60) for releasing said blocking means and for enabling said movable support means, together with said reservoir substrate, to be displaced towards said dispensing position; and
• said loading means include an elastically-deformable loading element (51; 51'; 1051') that co-operates with a cam surface (910; 910'; 1910), **characterized in that** said cam surface (910; 910'; 1910) comprises at least two different sloping surfaces, a first cam-surface portion that is adapted to deform and/or load said loading means, and a second cam-surface portion that co-operates with said loading means that are deformed and/or loaded, in the open position of the device.

2. A device according to claim 1, in which said elastically-deformable loading element comprises a compressible spring (51).

3. A device according to claim 2, in which a rod (810) is interposed between said spring (51) and said cam surface (910), with the displacement of the rod (810) against said cam surface (910) compressing and/or decompressing said spring (51).

4. A device according to claim 3, in which said cam surface (910) is formed on said movable support means (50) and presents a sloping first portion and a second portion of smaller slope, said rod (810) co-operating with said second cam-surface portion when the spring (51) is compressed, such that the force exerted by said rod (810) on said second cam-surface portion is substantially perpendicular to said second cam-surface portion.

5. A device according to claim 3 or claim 4, in which the end (811) of the rod (810) in contact with the cam surface (910) presents a profile that makes it easier to slide over said cam surface.

6. A device according to claim 5, in which said profile includes a surface that is rounded, and preferably spherical, at least in part.

7. A device according to claim 1, in which said elastically-deformable loading element comprises a flexible rod (51'; 1051').

8. A device according to claim 7, in which said rod (51'; 1051') is fastened on said movable support means (50), and includes a projection (51"; 1051") that co-operates with said cam surface (910'; 1910).

9. A device according to claim 8, in which said cam surface (910'; 1910) is formed by a groove that is made in said loading means (800), and in which said projection (51"; 1051") slides.

10. A device according to claim 9, in which said groove (910'; 1910) comprises a first groove portion for flexing said rod (51'; 1051') and a second groove portion, said rod (51'; 1051') co-operating with said second groove portion in the flexed state.

11. A device according to any preceding claim, in which said blocking means (100) comprise a rod (101) that is connected at one end to means (60) that are deformed under the effect of inhalation, and that includes at the other end a blocking element (110) that is adapted to co-operate with said movable support means (50).

12. A device according to claim 11, in which, in the open position of said at least one movable cover element (11, 12), said movable support means (50) exert a force on said blocking element (110).

13. A device according to claim 12, in which said rod (101) of the blocking means (100) includes a bearing zone (103) that is adapted to co-operate with a complementary zone (503) on said movable support means (50), such that, in the open position, said bearing zone (103) of the rod (101) exerts a force on said movable support means (50), with the direction of said force being substantially opposite to that of the force exerted by said movable support means (50) on said blocking element (110), so as to provide an open position that is stable.

14. A device according to claim 13, in which said bearing zone (103) is in the proximity of the end that is connected to the means (60) that are deformed by inhalation.

15. A device according to claim 13 or claim 14, in which the force exerted by said movable support means (50) on the blocking element (110) is greater than the force exerted by the bearing zone (103) of the rod (101) on said movable support means (50).
